(19) Europäisches Patentamt
European Patent Office
Office européen des brevets

(11) EP 1 497 288 B1

(12) EUROPÄISCHE PATENTSCHRIFT

(45) Veröffentlichungstag und Bekanntmachung des Hinweises auf die Patenterteilung:
**28.12.2005 Patentblatt 2005/52**

(21) Anmeldenummer: **03717295.4**

(22) Anmeldetag: **11.04.2003**

(51) Int Cl.[7]: **C07D 451/02**, A61K 31/46, A61P 11/06

(86) Internationale Anmeldenummer:
**PCT/EP2003/003765**

(87) Internationale Veröffentlichungsnummer:
**WO 2003/087096 (23.10.2003 Gazette 2003/43)**

(54) **NEUE ESTER HYDROXY-SUBSTITUIERTER STICKSTOFFHETEROCYCLEN ALS ANTAGONISTEN DES MUSKARINISCHEN M3 REZEPTORS, VERFAHREN ZU DEREN HERSTELLUNG SOWIE DEREN VERWENDUNG ALS ARZNEIMITTEL**

NOVEL ESTER HYDROXY-SUBSTITUTED NITROGEN HETEROCYCLES AS ANTAGONISTS OF THE MUSCARINIC M3 RECEPTOR, METHODS FOR THE PRODUCTION THEREOF, AND USE THEREOF AS A MEDICAMENT

NOUVEAUX ESTERS D'HETEROCYCLES D'AZOTE SUBSTITUES PAR UN HYDROXY UTILISES COMME ANTAGONISTES DU RECEPTEUR M3 MUSCARINIQUE, LEUR PROCEDE DE PRODUCTION ET LEUR UTILISATION COMME MEDICAMENTS

(84) Benannte Vertragsstaaten:
**AT BE BG CH CY CZ DE DK EE ES FI FR GB GR HU IE IT LI LU MC NL PT RO SE SI SK TR**
Benannte Erstreckungsstaaten:
**AL LT LV MK**

(30) Priorität: **13.04.2002 DE 10216339**

(43) Veröffentlichungstag der Anmeldung:
**19.01.2005 Patentblatt 2005/03**

(73) Patentinhaber: **Boehringer Ingelheim Pharma GmbH & Co.KG**
**55216 Ingelheim am Rhein (DE)**

(72) Erfinder:
- **PIEPER, Michael, P.**
**88400 BIBERACH (DE)**
- **POHL, Gerald**
**88400 BIBERACH (DE)**
- **SPECK, Georg**
**55218 INGELHEIM AM RHEIN (DE)**
- **GRAUERT, Matthias**
**88400 BIBERACH (DE)**
- **BREITFELDER, Steffen**
**88433 ASSMANNSHARDT (DE)**

(56) Entgegenhaltungen:
**WO-A-02/04402** **WO-A-92/16528**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 497 288 B1

Printed by Jouve, 75001 PARIS (FR)

## Beschreibung

Die vorliegende Erfindung betrifft neue Verbindungen der allgemeinen Formel 1

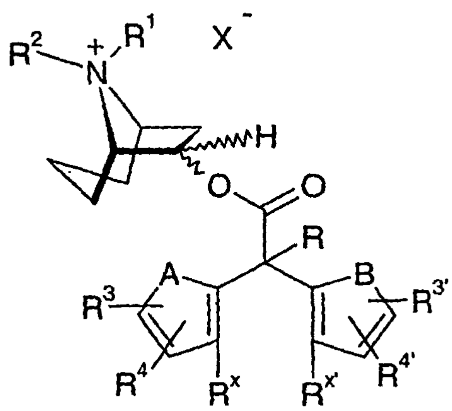

1

worin $X^-$ und die Reste A, B, R, $R^1$, $R^2$, $R^3$, $R^{3'}$, $R^4$, $R^{4'}$, $R^X$ und $R^{X'}$, die in den Ansprüchen und in der Beschreibung genannten Bedeutungen haben können, Verfahren zu deren Herstellung sowie deren Verwendung als Arzneimittel.

Strukturell verwandte anticholinergische Verbindungen sind in der WO 92/16528 beschrieben.

Beschreibung der Erfindung

Die vorliegende Erfindung betrifft Verbindungen der allgemeinen Formel 1

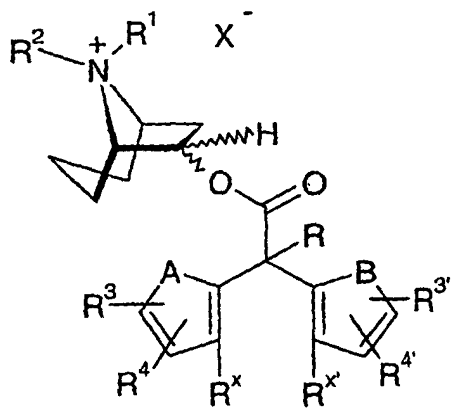

1

worin

| | |
|---|---|
| $X^-$ | ein einfach negativ geladenes Anion, vorzugsweise ein Anion ausgewählt aus der Gruppe bestehend aus Chlorid, Bromid, Iodid, Sulfat, Phosphat, Methansulfonat, Nitrat, Maleat, Acetat, Citrat, Fumarat, Tartrat, Oxalat, Succinat, Benzoat und p-Toluofsulfonat; |
| A und B | gleich oder verschieden, bevorzugt gleich, -O-, -S-, -NH-, $-CH_2-$, -CH=CH-, oder $-N(C_1-C_4-Alkyl)-$; |
| R | Wasserstoff, Hydroxy, $-C_1-C_4$-Alkyl, $-C_1-C_4$-Alkyloxy, $-C_1-C_4$-Alkylen-Halogen, $-O-C_1-C_4$-Alkylen-Halogen, $-C_1-C_4$-Alkylen-OH, $-CF_3$, $CHF_2$, $-C_1-C_4$-Alkylen-$C_1-C_4$-alkyloxy, $-O-COC_1-C_4$-Alkyl, $-O-COC_1-C_4$-Alkylen-Halogen, $-C_1-C_4$-Alkylen-$C_3-C_6$-Cycloalkyl, $-O-COCF_3$ oder Halogen; |
| $R^1$ und $R^2$ | gleich oder verschieden, $-C_1-C_5$-Alkyl, welches gegebenenfalls durch $-C_3-C_6$-Cycloalkyl, Hydroxy oder Halogen substituiert sein kann, oder<br>$R^1$ und $R^2$ gemeinsam eine $-C_3-C_5$-Alkylen-Brücke; |
| $R^3$, $R^4$, $R^{3'}$ und $R^{4'}$, | gleich oder verschieden, Wasserstoff, $C_1-C_4$-Alkyl, $C_1-C_4$-Alkyloxy, Hydroxy, $-CF_3$, $-CHF_2$, CN, |

| | |
|---|---|
| | $NO_2$ oder Halogen; |
| $R^X$ und $R^{X'}$ | gleich oder verschieden Wasserstoff, $C_1$-$C_4$-Alkyl, $C_1$-$C_4$-Alkyloxy, Hydroxy, $-CF_3$, $-CHF_2$, CN, $NO_2$ oder Halogen oder $R^X$ und $R^{X'}$ gemeinsam eine Einfachbindung oder eine verbrückende Gruppe ausgewählt aus den Brücken -O-, -S-, -NH-, $-CH_2$-, $-CH_2$-$CH_2$-, -N($C_1$-$C_4$-Alkyl)-, -CH($C_1$-$C_4$-Alkyl)- und -C($C_1$-$C_4$-Alkyl)$_2$-, bedeuten. |

Bevorzugt sind Verbindungen der allgemeinen Formel **1**, worin

| | |
|---|---|
| $X^-$ | ein einfach negativ geladenes Anion ausgewählt aus der Gruppe Chlorid, Bromid, 4-Toluolsulfonat und Methansulfonat, bevorzugt Bromid; |
| A und B | gleich oder verschieden, bevorzugt gleich, -O-, -S-, -NH- oder -CH=CH-; |
| R | Wasserstoff, Hydroxy, $-C_1$-$C_4$-Alkyl, $-C_1$-$C_4$-Alkyloxy, $-CF_3$, $-CHF_2$, Fluor, Chlor oder Brom; |
| $R^1$ und $R^2$ | gleich oder verschieden, $C_1$-$C_4$-Alkyl, welches gegebenenfalls durch Hydroxy, Fluor, Chlor oder Brom substituiert sein kann, oder $R^1$ und $R^2$ gemeinsam eine $-C_3$-$C_4$-Alkylen-Brücke; |
| $R^3$, $R^4$, $R^{3'}$ und $R^{4'}$, | gleich oder verschieden, Wasserstoff, $C_1$-$C_4$-Alkyl, $C_1$-$C_4$-Alkyloxy, Hydroxy, $-CF_3$, $-CHF_2$, CN, $NO_2$, Fluor, Chlor oder Brom; |
| $R^X$ und $R^{X'}$ | gleich oder verschieden Wasserstoff, $C_1$-$C_4$-Alkyl, $C_1$-$C_4$-Alkyloxy, Hydroxy, $-CF_3$, $-CHF_2$, CN, $NO_2$, Fluor, Chlor oder Brom oder $R^x$ und $R^{x'}$ gemeinsam eine Einfachbindung oder eine verbrückende Gruppe ausgewählt aus den Brücken -O-, -S-, -NH- und $-CH_2$- bedeuten. |

Besonders bevorzugt sind Verbindungen der allgemeinen Formel 1, worin

| | |
|---|---|
| $X^-$ | ein einfach negativ geladenes Anion ausgewählt aus der Gruppe Chlorid, Bromid und Methansulfonat, bevorzugt Bromid; |
| A und B | gleich oder verschieden, bevorzugt gleich, -S- oder -CH=CH-; |
| R | Wasserstoff, Hydroxy, Methyl, Ethyl, Methyloxy, Ethyloxy, $-CF_3$, oder Fluor; |
| $R^1$ und R2 | gleich oder verschieden, Methyl, Ethyl, $-CH_2F$ oder $-CH_2$-$CH_2F$, bevorzugt Methyl oder Ethyl; |
| $R^3$, $R^4$, $R^{3'}$ und $R^{4'}$, | gleich oder verschieden, Wasserstoff, Methyl, Methyloxy, $-CF_3$ oder Fluor; |
| $R^X$ und RX' | gleich oder verschieden Wasserstoff, Methyl, Methyloxy, $-CF_3$ oder Fluor oder $R^x$ und $R^{x'}$ gemeinsam eine Einfachbindung oder die verbrückende Gruppe -O- bedeuten. |

Erfindungsgemäß von besonderer Bedeutung sind Verbindungen der allgemeinen Formel **1**, worin

| | |
|---|---|
| $X^-$ | ein einfach negativ geladenes Anion ausgewählt aus der Gruppe Chlorid, Bromid und Methansulfonat, bevorzugt Bromid; |
| A und B | gleich oder verschieden, bevorzugt gleich, -S- oder -CH=CH-; |
| R | Wasserstoff, Hydroxy oder Methyl, bevorzugt Hydroxy; |
| $R^1$ und $R^2$ | gleich oder verschieden, Methyl oder Ethyl; |
| $R^3$, $R^4$, $R^{3'}$ und $R^{4'}$, | gleich oder verschieden, Wasserstoff, $-CF_3$ oder Fluor, bevorzugt Wasserstoff; |
| $R^x$ und $R^{x'}$ | gleich oder verschieden Wasserstoff, $-CF_3$ oder Fluor, bevorzugt Wasserstoff oder $R^x$ und $R^{x'}$ gemeinsam eine Einfachbindung oder die verbrückende Gruppe -O-, bevorzugt eine Einfachbindung bedeuten. |

Erfindungsgemäß bevorzugt sind ferner Verbindungen der allgemeinen Formel **1**, worin

| | |
|---|---|
| $X^-$ | Bromid; |
| A und B | gleich oder verschieden, bevorzugt gleich, -S- oder -CH=CH-; |
| R | Wasserstoff, Hydroxy oder Methyl, bevorzugt Hydroxy; |
| $R^1$ und $R^2$ | Methyl; |
| $R^3$, $R^4$, $R^{3'}$ und $R^{4'}$, | gleich oder verschieden, Wasserstoff oder Fluor, bevorzugt Wasserstoff; |
| $R^x$ und $R^{x'}$ | gleich oder verschieden Wasserstoff oder Fluor, bevorzugt Wasserstoff oder $R^x$ und $R^{x'}$ gemeinsam eine Einfachbindung oder die verbrückende Gruppe -O-, bevorzugt eine Einfachbindung bedeuten. |

Gegenstand der Erfindung sind die jeweiligen Verbindungen der Formel **1** gegebenenfalls in Form der einzelnen optischen Isomeren, Mischungen der einzelnen Enantiomeren oder Racemate sowie gegebenenfalls in Form ihrer pharmakologisch verträglichen Säureadditionssalze.

In den Verbindungen der allgemeinen Formel **1** können die Reste $R^3$, $R^4$, $R^{3'}$ und $R^{4'}$, sofern sie nicht Wasserstoff bedeuten, jeweils ortho, meta oder para bezüglich der Verknüpfung zur "-C- R"-Gruppe angeordnet sein. Sofern keiner der Reste $R^3$, $R^4$, $R^{3'}$ und $R^{4'}$ Wasserstoff bedeutet, sind $R^3$und $R^{3'}$ bevorzugt in para-Position und $R^4$ und $R^{4'}$ bevorzugt in ortho- oder meta-Position, besonders bevorzugt in meta-Position verknüpft. Sofern einer der Reste $R^3$ und $R^4$ und einer der Reste $R^{3'}$ und $R^{4'}$ Wasserstoff bedeutet, ist der jeweils andere Rest bevorzugt in meta- oder para-Position, besonders bevorzugt in para-Position verknüpft. Sofern keiner der Reste $R^3$, $R^4$, $R^{3'}$ und $R^{4'}$ Wasserstoff bedeutet sind erfindungsgemäß die Verbindungen der allgemeinen Formel **1** besonders bevorzugt, in denen die Reste $R^3$, $R^4$, $R^{3'}$ und $R^{4'}$ dieselbe Bedeutung aufweisen.

Von besonderer Bedeutung sind erfindungsgemäß die Verbindungen der allgemeinen Formel **1**, in denen A -CH=CH- und B -CH=CH- bedeuten.
Diese Verbindungen entsprechen der allgemeinen Formel **1'**.

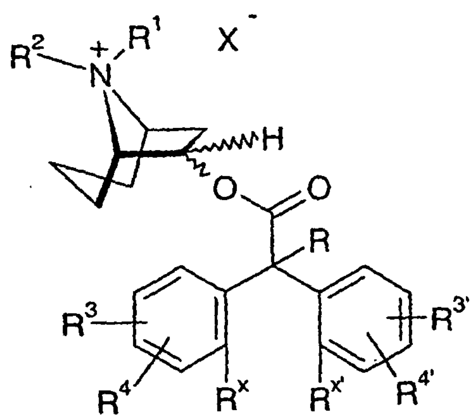

**1'**

worin $X^-$ und die Reste R, $R^1$, $R^2$, $R^3$, $R^{3'}$, $R^4$, $R^{4'}$, $R^x$ und $R^{x'}$, die vorstehend genannten Bedeutungen haben können.

Von erfindungsgemäß ebenfalls besonderer Bedeutung sind die Verbindungen der allgemeinen Formel **1**, in denen A -S- und B -S- bedeuten. Diese Verbindungen entsprechen der allgemeinen Formel **1''.**

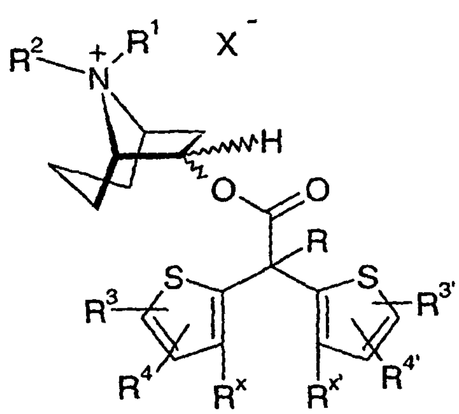

**1''**

worin $X^-$ und die Reste R, $R^1$, $R^2$, $R^3$, $R^{3'}$, $R^4$, $R^{4'}$, $R^x$ und $R^{x'}$, die vorstehend genannten Bedeutungen haben können.

Die nachfolgenden Verbindungen sind erfindungsgemäß von besonderer Bedeutung:

- Benzilsäure-tropane-6exo-yl-ester-Methobromid;
- 9-Hydroxy-fluoren-9-carbonsäuretropan-6exo-yl-ester-Methobromid;
- 2-Hydroxy-2,2-dithiophen-essigsäure-tropan-6exo-yl-ester-Methobromid;
- 9-Hydroxy-xanthen-9-carbonsäure-tropan-6exo-yl-ester-Methobromid;
- Benzilsäure-tropane-6endo-yl-ester-Methobromid;

- 9-Hydroxy-fluoren-9-carbonsäuretropan-6endo-yl-ester-Methobromid;
- 9-Hydroxy-xanthen-9-carbonsäure-tropan-6endo-ylester-Methobromid;
- 2-Hydroxy-2,2-dithiophen-essigsäure-tropan-6endo-yl-ester-Methobromid;

Als Alkylgruppen werden, soweit nicht anders angegeben, verzweigte und unverzweigte Alkylgruppen mit 1 bis 4 Kohlenstoffatomen bezeichnet. Beispielsweise werden genannt: Methyl, Ethyl, Propyl oder Butyl. Zur Bezeichnung der Gruppen Methyl, Ethyl, Propyl oder auch Butyl werden gegebenenfalls auch die Abkürzungen Me, Et, Prop oder Bu verwendet. Sofern nicht anders beschrieben, umfassen die Definitionen Propyl und Butyl alle denkbaren isomeren Formen der jeweiligen Reste. So umfaßt beispielsweise Propyl n-Propyl und iso-Propyl, Butyl umfaßt iso-Butyl, sec. Butyl und tert.-Butyl etc.

Als Cycloalkylgruppen werden, soweit nicht anders angegeben, alicyclische Gruppen mit 3 bis 6 Kohelnstoffatomen verstanden. Hierbei handelt es sich um die Gruppen Cyclopropyl, Cyclobutyl, Cyclopentyl und Cyclohexyl. Erfindungsgemäß von besonderer Bedeutung ist im Rahmen der vorliegenden Erfindung Cyclopropyl.

Als Alkylengruppen werden, soweit nicht anders angegeben, verzweigte und unverzweigte zweibindige Alkylbrücken mit 1 bis 4 Kohlenstoffatomen bezeichnet. Beispielsweise werden genannt: Methylen, Ethylen, Propylen oder Butylen.

Als Alkylen-Halogen-Gruppen werden, soweit nicht anders angegeben, verzweigte und unverzweigte zweibindige Alkylbrücken mit 1 bis 4 Kohlenstoffatomen bezeichnet, die ein-, zwei- oder dreifach, bevorzugt zweifach durch ein Halogen substituiert sind. Dementsprechend werden als Alkylen-OH-Gruppen, soweit nicht anders angegeben, verzweigte und unverzweigte zweibindige Alkylbrücken mit 1 bis 4 Kohlenstoffatomen bezeichnet, die ein-, zwei- oder dreifach, bevorzugt einfach durch ein Hydroxy substituiert sind.

Als Alkyloxygruppen werden, soweit nicht anders angegeben, verzweigte und unverzweigte Alkylgruppen mit 1 bis 4 Kohlenstoffatomen bezeichnet, die über ein Sauerstoffatom verknüpft sind. Beispielsweise werden genannt: Methylox, Ethyloxy, Propyloxy oder Butyloxy. Zur Bezeichnung der Gruppen Methyloxy, Ethyloxy, Propyloxy oder auch Butyloxy werden gegebenenfalls auch die Abkürzungen MeO-, EtO-, PropO- oder BuO- verwendet. Sofern nicht anders beschrieben, umfassen die Definitionen Propyloxy und Butyloxy alle denkbaren isomeren Formen der jeweiligen Reste. So umfaßt beispielsweise Propyloxy n-Propyloxy und iso-Propyloxy, Butyloxy umfaßt iso-Butyloxy, sec. Butyloxy und tert.-Butyloxy etc. Gegebenenfalls wird im Rahmen der vorliegenden Erfindung statt der Bezeichnung Alkyloxy auch die Bezeichnung Alkoxy verwendet. Zur Bezeichnung der Gruppen Methyloxy, Ethyloxy, Propyloxy oder auch Butyloxy gelangen dementsprechend gegebenenfalls auch die Ausdrücke Methoxy, Ethoxy, Propoxy oder Butoxy zur Anwendung.

Als Alkylen-alkyloxy-Gruppen werden, soweit nicht anders angegeben, verzweigte und unverzweigte zweibindige Alkylbrücken mit 1 bis 4 Kohlenstoffatomen bezeichnet, die ein-, zwei- oder dreifach, bevorzugt einfach durch eine Alkyloxygruppe substituiert sind.

Als -O-CO-Alkylgruppen werden, soweit nicht anders angegeben, verzweigte und unverzweigte Alkylgruppen mit 1 bis 4 Kohlenstoffatomen bezeichnet, die über eine Estergruppe verknüpft sind. Dabei sind die Alkylgruppen direkt an den Carbonylkohlenstoff der Estergruppe gebunden. In analoger Art und Weise ist die Bezeichnung -O-CO-Alkyl-Halogen-gruppe zu verstehen. Die Gruppe $-O-CO-CF_3$ steht für Trifluoracetat.

Halogen steht im Rahmen der vorliegenden Erfindung für Fluor, Chlor, Brom oder Jod. Sofern nicht gegenteilig angegeben, gelten Fluor und Brom als bevorzugte Halogene. Die Gruppe CO bezeichnet eine Carbonylgruppe.

Die Herstellung der erfindungsgemäßen Verbindungen kann, wie nachstehend erläutert, zum Teil in Analogie zu im Stand der Technik bereits bekannten Vorgehensweisen erfolgen (Schema 1).

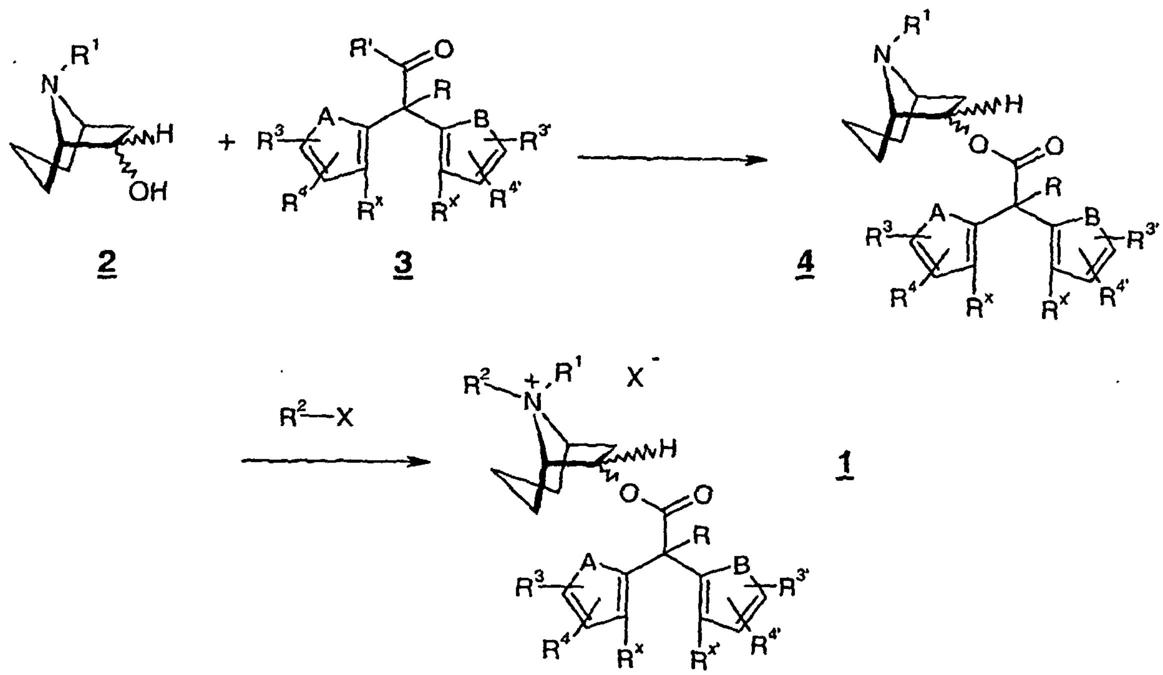


2 3 4

1

Schema 1:

Die carbonsäurederivate des formel 3 sind im Stand der Technik bekannt oder können nach im Stand der Technik bekannten Syntheseverfahren erhalten werde Sind lediglich geeignet substituierte Carbonsäuren im Stand der Technik bekannt, können die Verbindungen der Formel **3** auch direkt aus diesen durch Säure- oder Basen-katalysierte Veresterung mit den entsprechenden Alkoholen oder durch Halogenierung mit den entsprechenden Halogenierungsreagentien erhalten werden. Ausgehend von den Verbindungen der Formel **2** gelingt der Zugang zu den Estern der allgemeinen Formel **4** durch Umsetzung mit den Carbonsäurederivaten der Formel **3**, in denen R' beispielsweise für Chlor oder einen $C_1$-$C_4$-Alkyloxyrest steht. Im Falle von R' gleich $C_1$-$C_4$-Alkyloxy kann diese Umsetzung beispielsweise in einer Natriumschmelze bei erhöhter Temperatur, bevorzugt bei ca. 50-150°C, besonders bevorzugt bei etwa 90-100°C bei niedrigem Druck, bevorzugt bei unter 500mbar, besonders bevorzugt bei unter 75mbar durchgeführt werden. Alternativ dazu können statt der Derivate **3**, in denen R' $C_1$-$C_4$-Alkyloxy bedeutet auch die entsprechenden Säurechloride (R gleich Cl) eingesetzt werden.

Die so erhaltenen Verbindungen der Formel **4** lassen sich durch Umsetzung mit den Verbindungen $R^2$-X, in denen $R^2$ und X die vorstehend genannten Bedeutungen haben können, in die Zielverbindungen der Formel **1** überführen. Auch die Durchführung dieses Syntheseschritts kann in Analogie zu den in der WO 92/16528 offenbarten Synthesebeispielen erfolgen. In dem Fall in dem $R^1$ und $R^2$ gemeinsam eine Alkylenbrücke bilden, ist, wie für den Fachmann ersichtlich, die Zugabe des Reagenzes $R^2$-X nicht erforderlich. In diesem Fall weisen die Verbindungen der Formel **4** einen geeignet substituierten Rest $R^1$ (beispielsweise -$C_3$-$C_5$-Alkylen-Halogen) entsprechend der vorstehend genannten Definitionen auf und die Darstellung der Verbindungen der Formel **1** erfolgt durch intramolekulare Quarternierung des Amins.

Alternativ dazu sind die Verbindungen der Formel **4**, in denen R Halogen bedeutet auch auf dem in Schema 2 dargestellten Wege zugänglich.

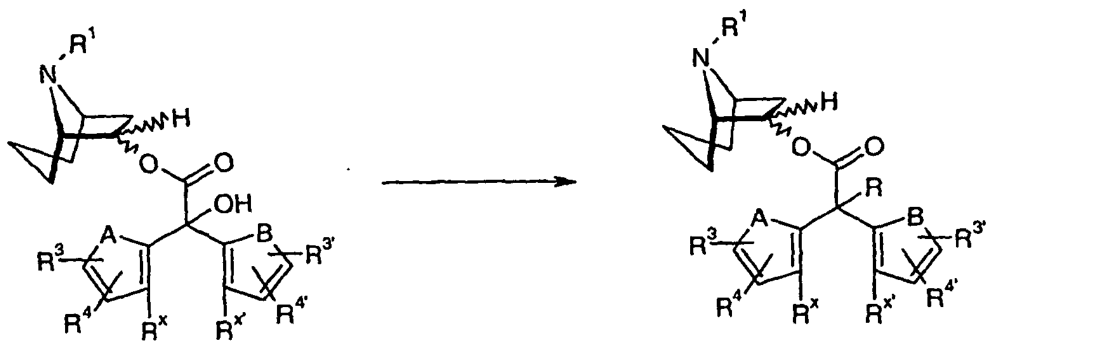

4 (mit R=OH) 4 (mit R = Halogen)

Schema 2:

Hierzu werden die Verbindungen der Formel 4 in denen R für Hydroxy steht unter Verwendung geeigneter Halogenierungsreagentien in die Verbindungen 4, in denen R Halogen bedeutet, überführt. Die Durchführung der nach Schema 2 durchzuführenden Halogenierungsreaktionen ist im Stand der Technik hinreichend bekannt.

Wie in Schema 1 ersichtlich, kommt den Zwischenprodukten der allgemeinen Formel 4 eine zentrale Bedeutung zu. Dementsprechend zielt ein weiterer Aspekt der vorliegenden Erfindung auf die Intermediate der Formel 4

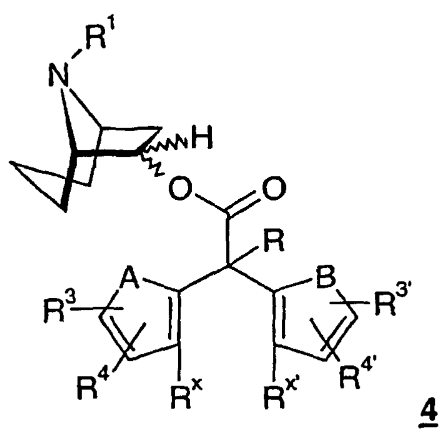

4

worin die Reste A, B, R, $R^1$, $R^3$, $R^{3'}$, $R^4$, $R^{4'}$, $R^x$ und $R^{x'}$ die vorstehend genannten Bedeutungen haben können, gegebenenfalls in Form ihrer Säureadditionssalze. Unter Säureadditionssalzen werden dabei Salze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat, bevorzugt Hydrochlorid, Hydrobromid, Hydrosulfat, Hydrophosphat, Hydrofumarat und Hydromethansulfonat verstanden.

Wie in den Verbindungen der allgemeinen Formel 1 können auch in den Verbindungen der allgemeinen Formel 4 die Reste $R^3$, $R^4$, $R^{3'}$ und $R^{4'}$, sofern sie nicht Wasserstoff bedeuten, jeweils ortho, meta oder para bezüglich der Verknüpfung zur "-C- R"-Gruppe angeordnet sein. Sofern keiner der Reste $R^3$, $R^4$, $R^{3'}$ und $R^{4'}$ Wasserstoff bedeutet, sind $R^3$und $R^{3'}$ bevorzugt in para-Position und $R^4$ und $R^{4'}$ bevorzugt in ortho- oder meta-Position, besonders bevorzugt in meta-Position verknüpft. Sofern einer der Reste $R^3$ und $R^4$ und einer der Reste R3' und $R^{4'}$ Wasserstoff bedeutet, ist der jeweils andere Rest bevorzugt in meta- oder para-Position, besonders bevorzugt in para-Position verknüpft. Sofern keiner der Reste $R^3$, $R^4$, $R^{3'}$ und $R^{4'}$ Wasserstoff bedeutet sind erfindungsgemäß die Verbindungen der allgemeinen Formel 4 besonders bevorzugt, in denen die Reste $R^3$, $R^4$, $R^{3'}$ und $R^{4'}$ dieselbe Bedeutung aufweisen.

Wie aus Schema 1 ersichtlich, dienen als Ausgangsprodukte für die Darstellung der Verbindungen der Formel 1 die Verbindungen der Formel **2.** Diese Verbindungen sind im Stand der Technik teilweise bekannt (Jones, J. Chem. Soc 1959, 615; D. E. Justice, THL 1995 4689-4692). Ein weiterer Aspekt der vorliegenden Erfindung betrifft allerdings die noch nicht bekannte Verwendung der Verbindungen der allgemeinen Formel 2

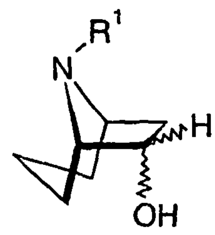

**2**

worin

$R^1$ Wasserstoff oder $-C_1-C_5$-Alkyl, welches gegebenenfalls durch $-C_3-C_6$-Cycloalkyl, Hydroxy oder Halogen substituiert sein kann, bedeutet, gegebenenfalls in Form Ihrer Säureadditionssalze, zur Herstellung der Verbindungen der allgemeinen Formel **4**.

Unter Säureadditionssalzen werden dabei Salze ausgewählt aus der Gruppe bestehend aus Hydrochlorid, Hydrobromid, Sulfat, Phosphat, Fumarat und Methansulfonat verstanden.

Bevorzugt ist die vorstehend genannte Verwendung von Verbindungen der allgemeinen Formel **2,** worin

$R^1$ Wasserstoff oder $C_1-C_4$-Alkyl, welches gegebenenfalls durch Hydroxy, Fluor, Chlor oder Brom substituiert sein kann, bedeutet, gegebenenfalls in Form Ihrer Säureadditionssalze.

Besonders bevorzugt ist die vorstehend genannte Verwendung von Verbindungen der allgemeinen Formel **2,** worin

$R^1$ Wasserstoff, Methyl, Ethyl, $-CH_2F$ oder $-CH_2-CH_2F$, bevorzugt Methyl oder Ethyl, bedeutet, gegebenenfalls in Form Ihrer Säureadditionssalze.

Erfindungsgemäß von besonderer Bedeutung ist die vorstehend genannte Verwendung von Verbindungen der allgemeinen Formel **2**, worin

$R^1$ Wasserstoff, Methyl oder Ethyl bedeutet, gegebenenfalls in Form Ihrer Säureadditionssalze.

Erfindungsgemäß bevorzugt ist ferner die vorstehend genannte Verwendung von Verbindungen der allgemeinen Formel **2,** worin

$R^1$ Wasserstoff oder Methyl bedeutet, gegebenenfalls in Form Ihrer Säureadditionssalze.

Ferner betrifft die vorliegende Erfindung die Verwendung der vorstehend genannten Verbindungen der allgemeinen Formel **2** als Ausgangsmaterial zur Herstellung der Verbindungen der allgemeinen Formel **1.**

Die nachstehend beschriebenen Synthesebeispiele dienen der weitergehenden Illustration der vorliegenden Erfindung. Sie sind allerdings nur als exemplarische Vorgehensweisen zur weitergehenden Erläuterung der Erfindung zu verstehen, ohne selbige auf den nachfolgend exemplarisch beschriebenen Gegenstand zu beschränken.

Darstellung der Basen **2a** und **2b:**

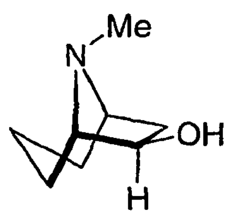

**2a**

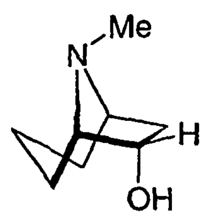

**2b**

Die Darstellung der Verbindungen der Formel **2a** und **2b** kann nach im Stand der Technik bekannten Methoden erfolgen (vgl. Jones, J. Chem. Soc 1959, 615; D. E. Justice, THL 1995 4689-4692).

**Beispiel 1:** Benzilsäure-tropane-6exo-yl-ester-Methobromid :

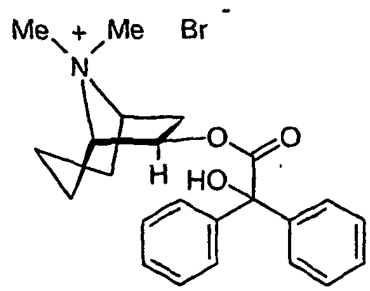

1.1.: Benzilsäuremethylester **3a:.**

90 g (0,394 mol) Benzilsäure werden in 900 ml Acetonitril gelöst und bei 5°C tropfenweise mit 109,6 g (0,72 mol) DBU versetzt. Nach Zugabe von 204,4 g (1,44 mol) Methyljodid wird 24 Stunden bei Raumtemperatur (etwa 20-23°C) gerührt. Die Lösung wird bis zum Rückstand eingedampft, der Rückstand in Diethylether aufgenommen und mit Wasser extrahiert. Die organische Phase wird mit 5%iger wässeriger Natriumcarbonatlösung und Wasser gewaschen, getrocknet und das Lösemittel abdestilliert. Die Reinigung des Produkts erfolgt durch Umkristallisation aus Cyclohexan. Ausbeute: 77,19 g weiße Kristalle (= 81% d. Th.) Schmp.: 74°-76° C.

1.2.: Benzilsäure-tropane-6exo-yl-ester **4a:**

1,2 g der Verbindung **2a** werden mit 2,42 g Benzylsäuremethylester **3a** und 10 mg Natrium versetzt und 1 h bei 70 mbar auf 100 °C erhitzt. Es wird abgekühlt und zur Zersetzung von überschüssigen Natrium 10ml Acetonitril zugegeben. Anschließend wird das Lösemittel unter vermindertem Druck abgestilliert. Der verbleibende Rückstand wird mit 100ml Toluol versetzt und 1x mit 100ml Wasser gewaschen. Die organische Phase wird getrocknet und das Lösemittel unter vermindertem Druck abdestilliert. Die Reinigung erfolgt mittels Chromatographie an Kieselgel ( ca 100ml Kieselgel; 150 ml Essigester, dann ca 500ml eines Lösemittelgemischs betstehend aus Dichlormethan 70 : Essigester 20 : Methanol 10). Ausbeute: 2,2 g (74%); Schmp.: 135°C.

1.3: Benzilsäure-tropane-6exo-yl-ester-Methobromid:

2,1 g der Verbindung **4a** werden in 40 mL Acetonitril und 40 mL Dichlormethan gelöst und mit 3,42 g einer 50%igen Lösung von Methylbromid in Acetonitril versetzt. Die Mischung wird 24 h bei RT (Raumtemperatur) gerührt und die ausgefallenen Kristalle abgesaugt und mit Diethylether gewaschen. Ausbeute: 2,4 g (90%); Schmp.: 264°C.

**Beispiel** 2: 9-Hydroxy-fluoren-9-carbonsäuretropan-6exo-yl-ester-Methobromid:

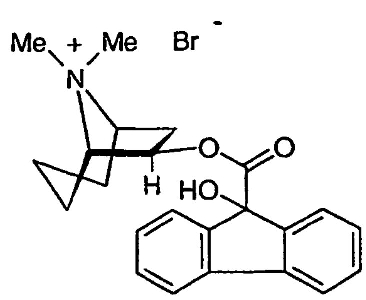

2.1.: 9-Hydroxy-fluoren-9-carbonsäuremethylester **3b:.**

50,4 g (0,223 mol) 9-Hydroxy-9-fluorencarbonsäure werden in 500 ml Methanol gelöst, mit 5 ml (0,089 mol) konz. Schwefelsäure versetzt und 1 Stunde unter Rückfluß gekocht. Nach Abkühlen werden 100 ml Natriumhydrogencarbonatlösung (ca. pH 8) zugegeben, und das Methanol unter vermindertem Druck weitestgehend abdestilliert. Der verbleibende Rückstand wird mit Dichlormethan und Wasser extrahiert, die organische Phase getrocknet und zur Trockene eingedampft.
Die Reinigung erfolgt durch Umkristallisation aus Ethylacetat.
Ausbeute: 50,0g (= 93% d. Th.)

2.2: 9-Hydroxy-fluoren-9-carbonsäuretropan-6exo-yl-ester **4b:**

1,05 g **2a,** 2,4 g Fluorenhydroxycarbonsäuremethylester **3b** und 10 mg Natrium werden 1 h bei 100°C und 70mbar gerührt. Es wird abgekühlt und zur Zersetzung von überschüssigen Natrium 10ml Acetonitril zugegeben. Anschließend wird das Lösemittel unter vermindertem Druck abdestilliert. Der Rückstand wird mit 100ml Toluol versetzt und 1 x mit 100ml Wasser gewaschen. Die organische Phase wird getrocknet, das Lösemittel unter vermindertem Druck abdestilliert und der Rückstand mittels Chromatographie gereinigt ( ca 100ml Kieselgel; 150 ml Essigester, dann ca 500ml eines Lösemittelgemischs bestehend aus Dichlormethan70 : Essigester 20 : Methanol 10). Ausbeute: 1,1 g (44%); Schmp. 154 °C.

2.3: 9-Hydroxy-fluoren-9-carbonsäuretropan-6exo-yl-ester-Methobromid:

1,1 g **4b** werden in 20 mL Acetonitril und 20 mL Dichlormethan gelöst, mit 1,82 g einer 50%igen Lösung von Methylbromid in Acetonitril versetzt und 24 h bei RT gerührt. Anschließend wird das Lösemittel unter vermindertem Druck abdestilliert und der Rückstand in 5 mL Methanol aufgenommen und mit 30 mL Aceton versetzt. Nach 2 h werden die ausgefallenen Kristalle abgesaugt. Ausbeute: 0,7 g (50%); Schmp.: > 300 °C (Zersetzung).

**Beispiel 3**: 2-Hydroxy-2,2-dithiophen-essigsäure-tropan-6exo-yl-ester-Methobromid:

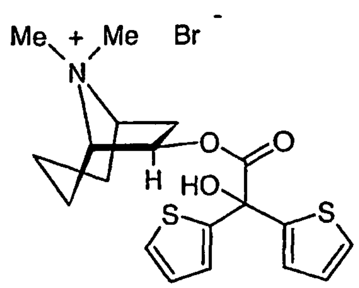

3.1: 2-Hydroxy-2,2-dithiophen-essigsäure-tropan-6exo-yl-ester **4c**:

1,05 g **2a,** 2,54 g Dithienylglykolsäuremethylester **3c** und 10 mg Natrium werden 1 h bei 100 °C und 70mbar gerührt. Es wird Abgekühlt und zur Zersetzung von überschüssigen Natrium 10ml Acetonitril zugegeben. Anschließend wird das Lösemittel unter vermindertem Druck abdestilliert. Der Rückstand wird mit 100ml Toluol versetzt und 1 x mit 100ml Wasser gewaschen. Die organische Phase wird getrocknet und das Lösemittel unter vermindertem Druck abdestilliert. Der Rückstand wird durch Chromatographie an Kieselgel gereinigt ( ca 100ml Kieselgel; 150 ml Essigester, dann ca 500ml eines Lösemittelgemischs bestehend aus Dichlormethan70 : Essigester 20 : Methanol 10). Ausbeute 0,9 g (35%);
Schmp.: 141 °C.

3.2: 2-Hydroxy-2,2-dithiophen-essigsäure-tropan-6exo-yl-ester-Methobromid:

0,9 g **4c** werden in 20 mL Acetonitril und 20 mL Dichlormethan gelöst, mit 1,42 g einer 50%igen Lösung von Methylbromid in Acetonitril versetzt und 24 h bei RT gerührt. Die ausgefallenen Kristalle werden abgesaugt und mit Ether gewaschen. Ausbeute: 0,9 g (79%); Schmp.: 220 °C (Zersetzung).

**Beispiel 4:** 9-Hydroxy-xanthen-9-carbonsäure-tropan-6exo-yl-ester-Methobromid :

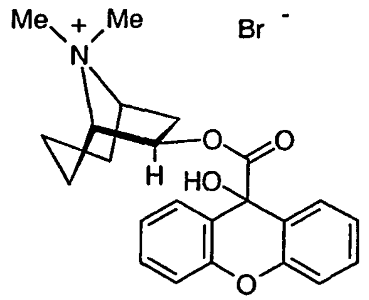

4.1.: 9-Hydroxy-xanthen-9-carbonsäuremethylester **3d:.**

a) Xanthen-9-carbonsäuremethylester:

Aus 21,75 g (0,95 mol) Natrium und 1500 ml Ethanol wird eine Natriumethylat-Lösung generiert. 214 g (0,95 mol) Xanthen-9-carbonsäure wird portionsweise in diese Lösung eingetragen und die erhaltene Suspension 1 Stunde bei Raumtemperatur nachgerührt. Danach wird der Feststoff abgetrennt, mit 1500 ml Diethylether gewaschen, die isolierten Kristalle in 1500 ml Dimethylformamid suspendiert und unter Rühren mit 126,73 ml (2,0 mol) Methyljodid versetzt. Die entstandene Lösung wird 24 Stunden bei Raumtemperatur stehengelassen, dann mit Wasser auf 6l Gesamtvolumen verdünnt, kristallisiert, abgesaugt, mit Wasser gewaschen und getrocknet. Ausbeute: 167 g weiße Kristalle 7 (= 74% d. Th.) Schmp.: 82° C.

b) 9-Hydroxy-xanthen-9-carbonsäuremethylester **3d:**

48,05 g (0,2 mol) Xanthen-9-carbonsäuremethylester werden in 1200 ml Tetrahydrofuran gelöst und bei 0° C mit 23,63 g (0,2 mol) Kalium-tert.butylat versetzt. Es wird anschließend 2 Stunden bei -10° bis -5° C Sauerstoff eingeleitet, danach mit 2 N wässeriger Salzsäure angesäuert und der größte Teil des Lösemittels destillativ entfernt. Der verbleibende Rückstand wird mit Ethylacetat und Wasser extrahiert, die organische Phase mit wässeriger $Na_2S_2O_5$-Lösung extrahiert, mit Wasser gewaschen, getrocknet und das Lösemittel abdetilliert. Die Reinigung des Produkts erfolgt durch Kristallisation aus Diisopropylether und Cyclohexan. Ausbeute: 11,10 g weiße Kristalle (= 22% d. Th.)

4.2: 9-Hydroxy-xanthen-9-carbonsäure- tropan-6exo-yl-ester **4d**:

0,705 g **2a**, 2,60 g 9-Hydroxy-xanthen-9-carbonsäuremethylester **3d** und 10 mg Natrium werden 2 h bei 100 °C und 70mbar gerührt. Es wird abgekühlt und zur Zersetzung von überschüssigen Natrium 10ml Acetonitril zugegeben. Anschließend wird das Lösemittel unter vermindertem Druck abdestilliert. Der Rückstand wird mit 100ml Toluol versetzt und 1 x mit 100ml Wasser gewaschen. Die organische Phase wird getrocknet und das Lösemittel unter vermindertem Druck abdestilliert. Der Rückstand wird durch Chromatographie gereinigt. ( ca 100ml Kieselgel; 150 ml Essigester, dann ca 500ml eines Lösemittelgemischs bestehend aus Dichlormethan70 : Essigester 20 : Methanol 10). Ausbeute 0,45 g (25%); amorpher Feststoff.

4.3: 9-Hydroxy-xanthen-9-carbonsäure-tropan-6exo-yl-ester-Methobromid

0,45 g **4d** werden in 4,5 mL Acetonitril gelöst, mit 0,50 g einer 50%igen Lösung von Methylbromid in Acetonitril versetzt und 24 h bei RT gerührt. Die ausgefallenen Kristalle werden abgesaugt und mit Ether gewaschen. Ausbeute 0,3 g (53%); Schmp.: 255°C.

Beispiel 5: Benzilsäure-tropane-6endo-yl-ester-Methobromid:

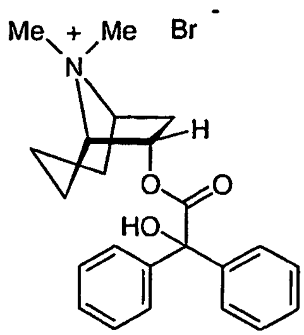

5.1.: Benzilsäuremethylester **3a:.**

Die Darstellung erfolgte gemäß Stufe 1.1.

5.2.: Benzilsäure-tropane-6endo-yl-ester **4e:**

0,6 g **2b** und 1,6 g Benzylsäuremethylester **3a** werden in 10 mL Toluol gelöst und mit 20 mg NaH versetzt. Man erwärmt auf 140°C wobei das entstehende Methanol abdestilliert. Nach 2 h wird abgekühlt, 20ml Wasser zugegeben, die organische Phase abgetrennt und 2x mit je 30 ml 1N Salzsäure extrahiert. Die Wasserphase wird mit Kaliumcarbonat versetzt und 2x mit je 30ml Dichlormethan extrahiert. Die vereinigten organischen Phasen werden getrocknet, das Lösemittel unter vermindertem Druck abdestilliert und der Rückstand aus Aceton mit etherischer HCl (gesättigt) kristallisiert. Ausbeute: 1,2 g; Schmp.: 265 °C.

5.3: Benzilsäure-tropane-6endo-yl-ester-Methobromid:

1,2 g **4e** werden in 50ml Wasser gelöst, mit Kaliumcarbonat versetzt und 2x mit je 30ml Dichlormethan extrahiert. Die organische Phase wird getrocknet und unter vermindertem Druck abdestilliert. Die Rückstand wird in 5 mL Acetonitril aufgenommen und mit 0,5 g einer 50%igen Lösung von Methylbromid in Acetonitril versetzt. Man läßt 24 bei RT rühren, saugt die ausgefallenen Kristalle ab und wäscht mit Aceton und Ether. Ausbeute 1,0 g (66%); Schmp.: 229 °C.

Beispiel 6: 9-Hydroxy-fluoren-9-carbonsäuretropan-6endo-yl-ester-Methobromid:

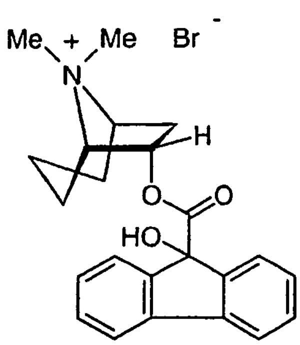

6.1.: 9-Hydroxy-fluoren-9-carbonsäuremethylester **3b:.**

Die Darstellung erfolgte entsprechend Stufe 2.1.

6.2: 9-Hydroxy-fluoren-9-carbonsäuretropan-6endo-yl-ester **4f:**

0,6 g **2b** und 1,6 g 9-Hydroxy-fluoren-9-carbonsäuremethylester **3b** werden in 10 mL Toluol gelöst und mit 20 mg NaH versetzt. Man erwärmt auf 140°C wobei das entstehende Methanol abdestilliert. Nach 2 h wird abgekühlt, 20ml Wasser zugegeben, die organische Phase abgetrennt und 2x mit je 30 ml 1N Salzsäure extrahiert. Die Wasserphase wird mit Kaliumcarbonat versetzt und 2x mit je 30ml Dichlormethan extrahiert. Die vereinigte organische Phase wird getrocknet, das Lösemittel unter vermindertem Druck abdestilliert und der Rückstand aus Aceton mit gesättigter etherischer HCl kristallisiert. Ausbeute: 1,2 g (73%); Schmp.: 261°C.

6.3: 9-Hydroxy-fluoren-9-carbonsäuretropan-6endo-yl-ester-Methobromid:

1,2 g **4f** werden in 50ml Wasser gelöst, mit Kaliumcarbonat versetzt und 2x mit je 30ml Dichlormethan extrahiert. Die organische Phase wird getrocknet und das Lösemittel unter vermindertem Druck abdestilliert. Der Rückstand wird in 5 mL Acetonitril aufgenommen und mit 0,5 g einer 50%igen Lösung von Methylbromid in Acetonitril versetzt. Man läßt 24 bei RT rühren, trennt die ausgefallene Kristalle ab und wäscht mit Aceton und Ether. Ausbeute: (70%).

**Beispiel 7**: 9-Hydroxy-xanthen-9-carbonsäure-tropan-6endo-ylester-Methobromid :

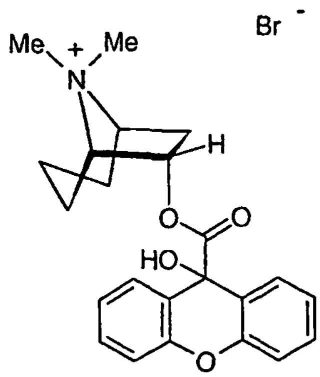

7.1.: 9-Hydroxy-xanthen-9-carbonsäuremethylester **3d**:.

Die Darstellung erfolgte gemäß Stufe 4.1.

7.2: 9-Hydroxy-xanthen-9-carbonsäure-tropan-6endo-ylester **4g**:

0,6 g **2b** und 1,6 g 9-Hydroxy-xanthen-9-carbonsäure-methylester **3d** werden in 10 mL Toluol gelöst und mit 20 mg NaH versetzt. Man erwärmt auf 140°C wobei das entstehende Methanol abdestilliert. Nach 2 h wird abgekühlt, 20ml Wasser zugegeben, die organische Phase abgetrennt und 2x mit je 30 ml 1 N Salzsäure extrahiert. Die Wasserphase wird mit Kaliumcarbonat versetzt und 2x mit je 30ml Dichlormethan extrahiert. Die vereinigte organische Phase wird getrocknet, das Lösemittel unter vermindertem Druck abdestilliert und der Rückstand aus Aceton mit gesättigter etherischenHCl kristallisiert. Ausbeute: 1,1 g (73%); Schmp.: 212 °C.

7.3: 9-Hydroxy-xanthen-9-carbonsäure-tropan-6endo-ylester-Methobromid:

1,1 g **4g** werden in 50ml Wasser gelöst, mit Kaliumcarbonat versetzt und 2x mit je 30ml Dichlormethan extrahiert. Die organische Phase wird getrocknet und das Lösemittel unter vermindertem Druck abdestilliert. Der Rückstand wird in 5 mL Acetonitril aufgenommen und mit 0,5 g einer 50%igen Lösung von Methylbromid in Acetonitril versetzt. Man läßt 24 bei RT rühren, trennt die ausgefallenen Kristalle ab und wäscht mit Aceton und Ether. Ausbeute: 1,0 g (72%); Schmp.: 250 °C.

**Beispiel 8:** 2-Hydroxy-2,2-dithiophen-essigsäure-tropan-6endo-yl-ester-Methobromid:

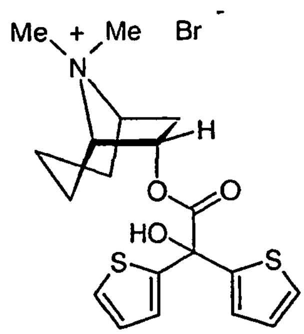

8.1: 2-Hydroxy-2,2-dithiophen-essigsäure-tropan-6endo-yl-ester **4h**:

0,6 g **2b** und 1,7 g Di-(2-thienyl)glykolsäuremethylester **3c** werden in 10 mL Toluol gelöst und mit 20 mg NaH versetzt. Man erwärmt auf 140°C wobei das entstehende Methanol abdestilliert. Nach 2 h wird abgekühlt, 20ml Wasser zugegeben, die organische Phase abgetrennt und 2x mit je 30 ml 1N Salzsäure extrahiert. Die Wasserphase wird mit Kaliumcarbonat versetzt und 2x mit je 30ml Dichlormethan extrahiert. Die vereinigte organische Phase wird getrocknet, das Lösemittel unter vermindertem Druck abdestilliert und der Rückstand aus Aceton mittels gesättigter etherischer HCl kristallisiert. Ausbeute: 1,1 g (65%);
Schmp.: 242 °C.

8.2: 2-Hydroxy-2,2-dithiophen-essigsäure-tropan-6endo-yl-ester-Methobromid:

1,1 g **4h** werden in 50ml Wasser gelöst, mit Kaliumcarbonat versetzt und 2x mit je 30ml Dichlormethan extrahiert. Die organische Phase wird getrocknet und das Lösemittel unter vermindertem Druck abdestilliert. Die Rückstand wird in 5 mL Acetonitril aufgenommen und mit 0,5 g einer 50%igen Lösung von Methylbromid in Acetonitril versetzt. Man läßt 24 bei RT rühren, trennt die ausgefallenen Kristalle ab und wäscht mit Aceton und Ether. Ausbeute: 1,1 g (79%); Schmp.: 218 °C.

Wie gefunden wurde, stellen die erfindungsgemäßen Verbindungen der Formel **1** Antagonisten des M3- Rezeptors (Muskarinischer Rezeptorsubtyp 3) dar. Die erfindungsgemäßen Verbindungen weisen bezüglich der Affinität zum M3-Rezeptor Ki-Werte von kleiner 100nM auf. Diese Werte wurde entsprechend der nachfolgend besschriebenen Vorgehensweise bestimmt.

Chemikalien

3H-NMS wurde von der Firma Amersham, Braunschweig, mit einer spezifischen Radioaktivität von 3071 GBq/ mmol (83 Ci/mmol) bezogen. Alle weiteren Raegantien wurden von Serva, Heidelberg und von Merck, Darmstadt erhalten.

Zellmembranen:

Wir setzten Zellmembranen von CHO Zellen (Chinese hamster ovary) ein, die mit den entsprechenden Genen der humanen muskarinischen Rezeptorsubtypen hm1 bis hm5 transfiziert waren (BONNER). Die Zellmembranen des gewünschten Subtypes wurden aufgetaut, resuspendiert per Hand mit einem Glas-Homogenisator und mit HEPES-Puffer auf eine Endkonzentration von 20-30 mg Protein/ml verdünnt.

Rezeptorbindungsstudien:

Der Bindungsassay wurde in einem Endvolumen von 1 ml ausgeführt und setzte sich zusammen aus 100 µl umarkierter Substanz in verschiedenen Konzentrationen, 100 µl Radioligand (3H-N-Methylscopolamin 2 nmol/L (3H-NMS), 200 µl Membranpräparation und 600 µl HEPES Puffer (20 mmol/L HEPES, 10 mmol/L MgCl2, 100 mmol/ L NaCl, mit 1 mol/L NaOH auf pH 7.4 eingestellt).

Die unspezifische Bindung ermittelten wir durch 10 µmol/L Atropin.
Die Inkubation von 45 min. erfolgte bei 37°C in 96-well Mikrotiterplatten (Beckman, Polystyrol, Nr. 267001) als Doppelbestimmung. Die Inkubation endete durch Filtration mittels Inotech Zellernter (Typ IH 110) über Whatman G-7 Filter. Die Filter wurden mit 3 ml eisgekühltem HEPES Puffer gewaschen und vor der Messung getrocknet.

Bestimmung der Radioaktivität:

Die Radioaktivität der Filtermatten wurden simultan mittels zweidimensionalem, digitalem Autoradiograph (Berthold, Wildbad, Typ 3052) gemessen.

Auswertung:

Die Ki-Werte berechneten wir mit impliziten Gleichungen, die direkt vom Massenwirkungsgesetz abgeleitet wurden, mit dem Modell für die 1 Rezeptor 2 Liganden Reaktion (SysFit - Software, SCHITTKOWSKI).

Literatur:

BONNER TI, New subtypes of muscarinic acetylcholine receptors
Trends Pharmacol. Sci. 10, Suppl.: 11-15 (1989); SCHITTKOWSKI K
Parameter estimation in systems of nonlinear equations Numer Math. 68: 129-142 (1994).

Die erfindungsgemäßen Verbindungen der Formel **1** zeichnen sich durch vielfältige Anwendungsmöglichkeiten auf therapeutischem Gebiet aus.
Hervorzuheben sind erfindungsgemäß solche Anwendungsmöglichkeiten, für welche die erfindungsgemäßen Verbindungen der Formel **1** aufgrund ihrer pharmazeutischen Wirksamkeit als Anticholinergikum bevorzugt zur Anwendung gelangen können.
Dies sind beispielsweise die Therapie von Asthma oder COPD (chronic obstructive pulmonary disease = chronisch obstruktive Lungenerkrankung). Die Verbindungen der allgemeinen Formel **1** können ferner zur Behandlung vagal bedingter Sinusbradykardien und zur Behandlung von Herz-Rhythmus-Störungen zum Einsatz gelangen. Generell lassen sich die erfindungsgemäßen Verbindungen ferner zur Behandlung von Spasmen beispielsweise im Gastrointestinaltrakt mit therapeutischem Nutzen einsetzen. Sie können ferner bei der Behandlung von Spasmen in harnableitenden Wegen sowie beispielsweise bei Menstruationsbeschwerden zum Einsatz gelangen.
Von den vorstehend beispielhaft aufgeführten Indikationsgebieten, kommt der Therapie von Asthma und COPD mittels der erfindungsgemäßen Verbindungen der Formel **1** eine besondere Bedeutung zu.

Die Verbindungen der allgemeinen Formel **1** können allein oder in Kombination mit anderen erfindungsgemäßen Wirkstoffen der Formel **1** zur Anwendung gelangen. Gegebenenfalls können die Verbindungen der allgemeinen Formel **1** auch in Kombination mit weiteren pharmakologisch aktiven Wirkstoffen eingesetzt werden. Es handelt sich hierbei insbesondere um Betamimetica, Antiallergika, Dopaminagonisten, PAF-Antagonisten, PDE IV-Inhibitoren, Leukotrien-Antagonisten, p38 Kinase-Inhibitoren, EGFR-Kinase-Hemmer und Corticosteroiden, sowie Wirkstoffkombinationen davon.

Als Beispiel für Betamimetika, die erfindungsgemäß mit den Verbindungen der Formel **1** als Kombination zum Einsatz kommen können, seien genannt Verbindungen, die ausgewählt sind aus der Gruppe bestehend aus Bambuterol, Bitolterol, Carbuterol, Clenbuterol, Fenoterol, Formoterol, Hexoprenalin, Ibuterol, Pirbuterol, Procaterol, Reproterol, Salmeterol, Sulfonterol, Terbutalin, Tolubuterol, 4-Hydroxy-7-[2-{[2-{[3-(2-phenylethoxy)propyl]sulfonyl}ethyl]-amino}ethyl]-2(3H)-benzothiazolon, 1-(2-Fluoro-4-hydroxyphenyl)-2-[4-(1-benzimidazolyl)-2-methyl-2-butylamino] ethanol, 1-[3-(4-Methoxybenzyl-amino)-4-hydroxyphenyl]-2-[4-(1-benzimidazolyl)-2-methyl-2-butylamino]ethanol, 1-[2H-5-Hydroxy-3-oxo-4H-1,4-benzoxazin-8-yl]-2-[3-(4-N,N-dimethylaminophenyl)-2-methyl-2-propylamino]ethanol, 1-[2H-5-Hydroxy-3-oxo-4H-1,4-benzoxazin-8-yl]-2-[3-(4-methoxyphenyl)-2-methyl-2-propylamino]ethanol, 1-[2H-5-Hydroxy-3-oxo-4H-1,4-benzoxazin-8-yl]-2-[3-(4-n-butyloxyphenyl)-2-methyl-2-propylamino]ethanol, 1-[2H-5-Hydroxy-3-oxo-4H-1,4-benzoxazin-8-yl]-2-{4-[3-(4-methoxyphenyl)-1,2,4-triazol-3-yl]-2-methyl-2-butylamino}ethanol, 5-Hydroxy-8-(1-hydroxy-2-isopropylaminobutyl)-2H-1,4-benzoxazin-3-(4H)-on, 1-(4-Amino-3-chloro-5-trifluormethylphenyl)-2-tert.-butylamino)ethanol und 1-(4-Ethoxycarbonylamino-3-cyano-5-fluorophenyl)-2-(tert.-butylamino)ethanol, gegebenenfalls in Form ihrer Racemate, ihrer Enantiomere, ihrer Diastereomere, sowie gegebenenfalls ihrer pharmakologisch unbedenklichen Säureadditionssalze, ihrer Solvate und/oder ihrer Hydrate. Besonders bevorzugt gelangen als Betamimetika solche Wirkstoffe in Kombination mit den erfindungsgemäßen Verbindungen der Formel **1** zur Anwendung, die ausgewählt sind aus der Gruppe bestehend aus Fenoterol, Formoterol, Salmeterol, 1-[3-(4-Methoxybenzyl-amino)-4-hydroxyphenyl]-2-[4-(1-benzimidazolyl)-2-methyl-2-butylamino]ethanol, 1-[2H-5-Hydroxy-3-oxo-4H-1,4-benzoxazin-8-yl]-2-[3-(4-N,N-dimethylaminophenyl)-2-methyl-2-propylamino]ethanol, 1-[2H-5-Hydroxy-3-oxo-4H-1,4-benzoxazin-8-yl]-2-[3-(4-methoxyphenyl)-2-methyl-2-propylamino]ethanol, 1-[2H-5-Hydroxy-3-oxo-4H-1,4-benzoxazin-8-yl]-2-[3-(4-n-butyloxyphenyl)-2-methyl-2-propylamino]ethanol, 1-[2H-5-Hydroxy-3-oxo-4H-1,4-benzoxazin-8-yl]-2-{4-[3-(4-methoxyphenyl)-1,2,4-triazol-3-yl]-2-methyl-2-butylamino}ethanol, gegebenenfalls in Form ihrer Racemate, ihrer Enantiomere, ihrer Diastereomere, sowie gegebenenfalls ihrer pharmakologisch unbedenklichen Säureadditionssalze und Hydrate. Von den vorstehend genannten Betamimetika kommt hierbei den Verbindungen Formoterol und Salmeterol gegebenenfalls in Form ihrer Racemate, ihrer Enantiomere, ihrer Diastereomere, sowie gegebenenfalls ihrer pharmakologisch unbedenklichen Säureadditionssalze und Hydrate besondere Bedeutung zu. Erfindungsgemäß bevorzugt sind die Säureadditionssalze der Betamimetika beispielsweise ausgewählt aus der Gruppe bestehend aus Hydrochlorid, Hydrobromid, Sulfat, Phosphat, Fumarat, Methansulfonat und Xinafoat. Besonders bevorzugt sind die Salze im Falle des Salmeterols ausgewählt aus Hydrochlorid, Sulfat und Xinafoat, von denen das Xinafoate besonders bevorzugt ist. Besonders bevorzugt sind die Salze im Falle des Formoterols ausgewählt aus Hydrochlorid, Sulfat und Fumarat, von denen das Hydrochlorid und Fumarat besonders bevorzugt sind. Erfindungsgemäß von herausragender Bedeutung ist Formoterolfumarat.

Im Rahmen der vorliegenden Erfindung werden unter Corticosteroiden, die gegebenenfalls in Kombination mit den Verbindungen der Formel **1** zum Einsatz gelangen können, Verbindungen verstanden, die ausgewählt sind aus der Gruppe bestehend aus Flunisolide, Beclomethasone, Triamcinolone, Budesonid, Fluticasone, Mometasone, Ciclesonide, Rofleponide, GW 215864, KSR 592, ST-126 und Dexametasone. Bevorzugt sind im Rahmen der vorliegenden Erfindung die Corticosteroide ausgewählt aus der Gruppe bestehend aus Flunisolide, Beclomethasone, Triamcinolone, Budesonid, Fluticasone, Mometasone, Ciclesonide und Dexametasone, wobei hier dem Budesonid, Fluticasone, Mometasone und Ciclesonide, insbesondere dem Budesonid und dem Fluticason eine besondere Bedeutung zukommt. Gegebenfalls wird im Rahmen der vorliegenden Patentanmeldung statt der Bezeichnung Corticosteroide auch nur die Bezeichnung Steroide verwendet. Eine Bezugnahme auf Steroide schließt im Rahmen der vorliegenden Erfindung eine Bezugnahme auf Salze oder Derivate, die von den Steroiden gebildet werden können, mit ein. Als mögliche Salze oder Derivate werden beispielsweise genannt: Natriumsalze, Sulfobenzoate, Phosphate, Isonicotinate, Acetate, Propionate, Dihydrogenphosphate, Palmitate, Pivalate oder Furoate. Gegebenenfalls können die Corticosteroide auch in Form ihrer Hydrate vorliegen.

Als Beispiel für PDE-IV-Inhibitoren, die erfindungsgemäß mit der Verbindung der Formel **1** als Kombination zum Einsatz kommen können, seien genannt Verbindungen, die ausgewählt sind aus der Gruppe bestehend aus Enprofylline, Roflumilast, Ariflo, Bay-198004, CP-325,366, BY343, D-4396 (Sch-351591), V-11294A und AWD-12-281. Bevorzugte PDE-IV-Inhibitoren sind ausgewählt aus der Gruppe bestehend aus Enprofylline, Roflumilast, Ariflo und AWD-12-281, wobei AWD-12-281 als Kombinationspartner mit der erfindungsgemäßen Verbindung der Formel **1** besonders bevorzugt ist. Eine Bezugnahme auf die vorstehend genannten PDE-IV-Inhibitoren schließt im Rahmen der vorliegenden Erfindung eine Bezugnahme auf deren gegebenenfalls existierende pharmakologisch verträgliche Säureadditionssalze ein. Unter den physiologisch verträglichen Säureadditionssalzen, die von den vorstehend genannten PDE-IV-Inhibitoren gebildet werden können, werden erfindungsgemäß pharmazeutisch verträgliche Salze verstanden, die ausgewählt aus den Salzen der Salzsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure oder Maleinsäure sind. Erfindungsgemäß bevorzugt sind in diesem Zusammenhang die Salze ausgewählt aus der Gruppe bestehend aus Acetat, Hydrochlorid, Hydrobromid, Sulfat, Phosphat und Methansulfonat.

Im Rahmen der vorliegenden Erfindung werden unter Dopamin-Agonisten, die gegebenenfalls in Kombination mit den Verbindungen der Formel **1** zum Einsatz gelangen können, Verbindungen verstanden, die ausgewählt sind aus der Gruppe bestehend aus Bromocriptin, Cabergolin, Alpha-Dihydroergocryptin, Lisurid, Pergolid, Pramipexol, Roxindol, Ropinirol, Talipexol, Tergurid und Viozan. Bevorzugt werden im Rahmen der vorliegenden Erfindung Dopamin-Agonisten als Kombinationspartner mit den Verbindungen der Formel **1** eingesetzt, die ausgewählt sind aus der Gruppe bestehend aus Pramipexol, Talipexol und Viozan, wobei Pramipexol eine besondere Bedeutung zukommt. Eine Bezugnahme auf die vorstehend genannten Dopamin-Agonisten schließt im Rahmen der vorliegenden Erfindung eine Bezugnahme auf deren gegebenenfalls existierende pharmakologisch verträgliche Säureadditionssalze und gegebenenfalls deren Hydrate ein. Unter den physiologisch verträglichen Säureadditionssalzen, die von den vorstehend genannten Dopaminagonisten gebildet werden können, werden beispielsweise pharmazeutisch verträgliche Salze verstanden, die ausgewählt aus den Salzen der Salzsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure und Maleinsäure sind.

Als Beispiel für Antiallergika, die erfindungsgemäß mit den Verbindungen der Formel **1** als Kombination zum Einsatz kommen können, seien genannt Epinastin, Cetirizin, Azelastin, Fexofenadin, Levocabastin, Loratadin, Mizolastin, Ketotifen, Emedastin, Dimetinden, Clemastin, Bamipin, Cexchlorpheniramin, Pheniramin, Doxylamin, Chlorphenoxamin, Dimenhydrinat, Diphenhydramin, Promethazin, Ebastin, Desloratidin und Meclozin. Bevorzugte Antiallergika, die im Rahmen der vorliegenden Erfindung in Kombination mit den erfindungsgemäßen Verbindungen der Formel **1** zum Einsatz gelangen können, sind ausgewählt aus der Gruppe bestehend aus Epinastin, Cetirizin, Azelastin, Fexofenadin, Levocabastin, Loratadin, Ebastin, Desloratidin und Mizolastin wobei Epinastin und Desloratidin besonders bevorzugt sind. Eine Bezugnahme auf die vorstehend genannten Antiallergika schließt im Rahmen der vorliegenden Erfindung eine Bezugnahme auf deren gegebenenfalls existierende pharmakologisch verträgliche Säureadditionssalze ein.

Als Beispiel für PAF-Antagonisten, die erfindungsgemäß mit den Verbindungen der Formel **1** als Kombination zum Einsatz kommen können seien genannt 4-(2-Chlorphenyl)-9-methyl-2-[3(4-morpholinyl)-3-propanon-1-yl]-6H-thieno-[3,2-f] [1,2,4]triazolo[4,3-a][1,4]diazepin, 6-(2-Chlorphenyl)-8,9-dihydro-1-methyl-8-[(4-morpholinyl)carbonyl]-4H,7H-cyclopenta-[4,5]thieno-[3,2-f][1,2,4]triazolo[4,3-a][1,4]diazepin.

Als Beispiel für EGFR-Kinase-Hemmer, die mit den erfindungsgemäßen Verbindungen der Formel **1** als Kombination zum Einsatz kommen können, seien als besonders bevorzugt genannt (4-[(3-Chlor-4-fluor-phenyl)amino]-7-[4-((R)-6-methyl-2-oxo-morpholin-4-yl)-butyloxy]-6-[(vinylcarbonyl)amino]-chinazolin, 4-[(3-Chlor-4-fluorphenyl)amino]-7-[4-((S)-6-methyl-2-oxo-morpholin-4-yl)-butyloxy]-6-[(vinylcarbonyl)amino]-chinazolin, 4-[(3-Chlor-4-fluorphenyl)amino]-7-(2-{4-[(S)-(2-oxo-tetrahydrofuran-5-yl)carbonyl]-piperazin-1-yl}-ethoxy)-6-[(vinylcarbonyl)amino]-chinazolin, 4-[(3-Chlor-4-fluor-phenyl)amino]-7-[2-((S)-6-methyl-2-oxo-morpholin-4-yl)-ethoxy]-6-[(vinylcarbonyl)amino]-chinazolin, 4-[(3-Chlor-4-fluorphenyl)amino]-6-[(4-{N-[2-(ethoxycarbonyl)-ethyl]-N-[(ethoxycarbonyl)methyl]amino}-1-oxo-2-buten-1-yl)amino]-7-cyclopropylmethoxy-chinazolin, 4-[(R)-(1-Phenyl-ethyl)amino]-6-{[4-(morpholin-4-yl)-1-oxo-2-buten-1-yl]amino}-7-cyclopropylmethoxy-chinazolin und 4-[(3-Chlor-4-fluorphenyl)amino]-6-[3-(morpholin-4-yl)-propyloxy]-7-methoxychinazolin. Eine Bezugnahme auf die vorstehend genannten EGFR-Kinase-Hemmer schließt im Rahmen der vorliegenden Erfindung eine Bezugnahme auf deren gegebenenfalls existierende pharmakologisch verträgliche Säureadditionssalze ein. Unter den physiologischbzw. pharmakologisch verträglichen Säureadditionssalzen, die von den EGFR-Kinase-Hemmern gebildet werden können, werden erfindungsgemäß pharmazeutisch verträgliche Salze verstanden, die ausgewählt aus den Salzen der Salzsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure oder Maleinsäure sind. Erfindungsgemäß bevorzugt sind die Salze der EGFR-Kinase-Hemmer ausgewählt aus den Salzen der Essigsäure, Salzsäure, Bromwasserstoffsäure, Schwefelsäure, Phosphorsäure und Methansulfonsäure.

Als Beispiel für p38 Kinase-Hemmer, die mit den erfindungsgemäßen Verbindungen der Formel **1** als Kombination zum Einsatz kommen können, seien als besonders bevorzugt genannt 1-[5-*tert*-Butyl-2-p-tolyl-2H-pyrazol-3-yl]-3-[4-(2-morpholin-4-yl-ethoxy)naphthalin-1-yl]-harnstoff; 1-[5-*tert*-Butyl-2-*p*-tolyl-2H-pyrazol-3-yl]-3-[4-(2-(1-oxothiomorpholin-4-yl)ethoxy)naphthalin-1-yl]-harnstoff;1-[5-*tert*-butyl-2-(2-methylpyridin-5-yl)-2H-pyrazol-3-yl]-3-[4-(2-pyridin-4-yl-ethoxy)naphthalin-1-yl]-harnstoff; 1-[5-*tert*-butyl- 2-(2-methoxypyridin-5-yl)-2H-pyrazol-3-yl]-3-[4-(2-morpholin-4-yl-ethoxy)naphthalin-1-yl]-harnstoff oder 1-[5-*tert*-butyl-2-methyl-2H-pyrazol-3-yl]-3-[4-(2-morpholin-4-yl-ethoxy)naphthalen-1-yl]-harnstoff. Eine Bezugnahme auf die vorstehend genannten p38-Kinase-Hemmer schließt im Rahmen der vorliegenden Erfindung eine Bezugnahme auf deren gegebenenfalls existierende pharmakologisch verträgliche Säureadditionssalze ein. Unter den physiologisch bzw. pharmakologisch verträglichen Säureadditionssalzen, die von den p38-Kinase-hemmern gebildet werden können, werden erfindungsgemäß pharmazeutisch verträgliche Salze verstanden, die ausgewählt aus den Salzen der Salzsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure oder Maleinsäure sind.

Werden die Verbindungen der Formel **1** in Kombination mit anderen Wirkstoffen eingesetzt, ist von den vorstehend genannten Verbindungsklassen die Kombination mit Steroiden, PDE IV-inhibitoren oder Betamimetika besonders bevorzugt. Der Kombination mit Betamimetika, insbesondere mit langwirksamen Betamimetika kommt dabei eine besondere Bedeutung zu. Als besonders bevorzugt ist die Kombination der erfindungsgemäßen Verbindungen der Formel **1** mit Salmeterol oder Formoterol anzusehen.

Geeignete Anwendungsformen zur Applikation der Verbindungen der Formel **1** sind beispielsweise Tabletten, Kapseln, Zäpfchen, Lösungen etc.

Erfindungsgemäß von besonderer Bedeutung ist (insbesondere bei der Behandlung von Asthma oder COPD) die inhalative Applikation der erfindungsgemäßen Verbindungen. Der Anteil der pharmazeutisch wirksamen Verbindung(en) sollte jeweils im Bereich von 0,05 bis 90 Gew.-%, bevorzugt 0,1 bis 50 Gew.-% der Gesamtzusammensetzung liegen. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen, wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Lösungen werden in üblicher Weise, z.B. unter Zusatz von Isotonantien, Konservierungsmitteln, wie p-Hydroxybenzoaten, oder Stabilisatoren, wie Alkalisalzen der Ethylendiamintetraessigsäure, gegebenenfalls unter Verwendung von Emulgiermitteln und /oder Dispergiermitteln, wobei beispielsweise bei der Verwendung von Wasser als Verdünnungsmittel gegebenenfalls organische Lösemittel als Lösevermittler bzw. Hilflösemittel eingesetzt werden können, hergestellt und in Injektionsflaschen oder Ampullen oder Infusionsflaschen abgefüllt.

Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.

Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Als Hilfsstoffe seien beispielsweise Wasser, pharmazeutisch unbedenkliche organische Lösemittel, wie Paraffine (z. B. Erdölfraktionen), Öle pflanzlichen Ursprungs (z.B. Erdnuß- oder Sesamöl), mono- oder polyfunktionelle Alkohole (z.B. Ethanol oder Glycerin), Trägerstoffe wie z.B. natürliche Gesteinsmehle (z.B. Kaoline, Tonerden, Talkum, Kreide) synthetische Gesteinsmehle (z.B. hochdisperse Kieselsäure und Silikate), Zucker (z.B. Rohr-, Milch- und Traubenzukker) Emulgiermittel (z.B. Lignin, Sufitablaugen, Methylcellulose, Stärke und Polyvinylpyrrolidon) und Gleitmittel (z.B. Magnesiumstearat, Talkum, Stearinsäure und Natriumlaurylsulfat) erwähnt.

Im Falle der oralen Anwendung können die Tabletten selbstverständlich außer den genannten Trägerstoffen auch Zusätze, wie z.B. Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Bei der erfindungsgemäß bevorzugten Applikation der Verbindungen der Formel **1** zur Therapie von Asthma oder COPD werden besonders bevorzugt inhalativ applizierbare Darreichungsformen bzw. pharmazeutische Formulierungen eingesetzt. Als inhalierbare Darreichungsformen kommen Inhalationspulver, treibgashaltige Dosieraerosole oder treibgasfreie Inhalationslösungen in Betracht. Im Rahmen der vorliegenden Erfindung sind von dem Begriff treibgasfreie Inhalationslösungen auch Konzentrate oder sterile, gebrauchsfertige Inhalationslösungen umfaßt. Die im Rahmen der vorliegenden Erfindung einsetzbaren Darreichungsformen werden im nachfolgenden Teil der Beschreibung detailliert beschrieben.

Erindungsgemäß einsetzbare Inhalationspulver können **1** entweder allein oder im Gemisch mit geeigneten physiologisch unbedenkliche Hilfsstoffen enthalten.

Sind die Wirkstoffe **1** im Gemisch mit physiologisch unbedenklichen Hilfsstoffen enthalten, können zur Darstellung dieser erfindungsgemäßen Inhalationspulver die folgenden physiologisch unbedenklichen Hilfsstoffe zur Anwendung gelangen: Monosaccharide (z.B. Glucose oder Arabinose), Disaccharide (z.B. Lactose, Saccharose, Maltose), Oligo- und Polysaccharide (z.B. Dextrane), Polyalkohole (z.B. Sorbit, Mannit, Xylit), Salze (z.B. Natriumchlorid, Calciumcarbonat) oder Mischungen dieser Hilfsstoffe miteinander. Bevorzugt gelangen Mono- oder Disaccharide zur Anwendung, wobei die Verwendung von Lactose oder Glucose, insbesondere, aber nicht ausschließlich in Form ihrer Hydrate, bevorzugt ist. Als besonders bevorzugt im Sinne der Erfindung gelangt Lactose, höchst bevorzugt Lactosemonohydrat als Hilfsstoff zur Anwendung.

Die Hilfsstoffe weisen im Rahmen der erfindungsgemäßen Inhalationspulver eine maximale mittlere Teilchengröße von bis zu 250µm, bevorzugt zwischen 10 und 150µm, besonders bevorzugt zwischen 15 und 80µm auf. Gegebenenfalls kann es sinnvoll erscheinen, den vorstehend genannten Hilfststoffen feinere Hilfsstofffraktionen mit einer mittleren Teilchengröße von 1 bis 9µm beizumischen. Letztgenannte feinere Hilfsstoffe sind ebenfalls ausgewählt aus der vorstehend genannten Gruppe an einsetzbaren Hilfsstoffen. Schließlich wird zur Herstellung der erfindungsgemäßen Inhaltionspulver mikronisierter Wirkstoff **1,** vorzugsweise mit einer mittleren Teilchengröße von 0,5 bis 10µm, besonders bevorzugt von **1** bis 5µm, der Hilfsstoffmischung beigemischt. Verfahren zur Herstellung der erfindungsgemäßen Inhaltionspulver durch Mahlen und Mikronisieren sowie durch abschließendes Mischen der Bestandteile sind aus dem Stand der Technik bekannt. Die erfindungsgemäßen Inhalationspulver können mittels aus dem Stand der Technik bekannten Inhalatoren appliziert werden.

Erfindungsgemäße treibgashaltige Inhalationsaerosole können **1** im Treibgas gelöst oder in dispergierter Form enthalten. Hierbei können **1** in getrennten Darreichungsformen oder in einer gemeinsamen Darreichungsform enthalten sein, wobei **1** entweder beide gelöst, beide dispergiert oder jeweils nur eine Komponente gelöst und die andere dispergiert enthalten sein können.

Die zur Herstellung der Inhalationsaerosole einsetzbaren Treibgase sind aus dem Stand der Technik bekannt. Geeignete Treibgase sind ausgewählt aus der Gruppe bestehend aus Kohlenwasserstoffen wie n-Propan, n-Butan oder Isobutan und Halogenkohlenwasserstoffen wie fluorierten Derivaten des Methans, Ethans, Propans, Butans, Cyclopropans oder Cyclobutans. Die vorstehend genannten Treibgase können dabei allein oder in Mischungen derselben zur Verwendnung kommen. Besonders bevorzugte Treibgase sind halogenierte Alkanderivate ausgewählt aus TG134a und TG227 und Mischungen derselben.

Die treibgashaltigen Inhalationsaerosole können ferner weitere Bestandteile wie Kosolventien, Stabilisatoren, oberflächenaktive Mittel (surfactants), Antioxidantien, Schmiermittel sowie Mittel zur Einstellung des pH-Werts enthalten. All diese Bestandteile sind im Stand der Technik bekannt.

Die vorstehend genannten treibgashaltigen Inhalationaerosole können mittels im Stand der Technik bekannten Inhalatoren (MDIs = metered dose inhalers) appliziert werden.

Ferner kann die Applikation der erfindungsgemäßen Wirkstoffe **1** in Form von treibgasfreien Inhalationslösungen und Inhalationssuspensionen. Als Lösungsmittel kommen hierzu wässrige oder alkoholische, bevorzugt ethanolische Lösungen in Betracht. Das Lösungsmittel kann ausschließlich Wasser sein oder es ist ein Gemisch aus Wasser und Ethanol. Der relative Anteil an Ethanol gegenüber Wasser ist nicht begrenzt, bevorzugt liegt die maximale Grenze jedoch bei bis 70 Volumenprozent, insbesondere bei bis zu 60 Volumenprozent und besonders bevorzugt bei bis zu 30 Volumenprozent. Die restlichen Volumenprozente werden von Wasser aufgefüllt. Die **1** enthaltenden Lösungen oder Suspensionen werden mit geeigneten Säuren auf einen pH-Wert von 2 bis 7, bevorzugt von 2 bis 5 eingestellt. Zur Einstellung dieses pH-Werts können Säuren ausgewählt aus anorganischen oder organischen Säuren Verwendung finden. Beispiele für besonders geeignete anorganische Säuren sind Salzsäure, Bromwasserstoffsäure, Salpetersäure, Schwefelsäure und/oder Phosphorsäure. Beispiele für besonders geeignete organische Säuren sind: Ascorbinsäure, Zitronensäure, Äpfelsäure, Weinsäure, Maleinsäure, Bernsteinsäure, Fumarsäure, Essigsäure, Ameisensäure und/oder Propionsäure und andere. Bevorzugte anorganische Säuren sind Salzsäure, Schwefelsäure. Es können auch die Säuren verwendet werden, die bereits mit einem der Wirkstoffe ein Säureadditionssalz bilden. Unter den organischen Säuren sind Ascorbinsäure, Fumarsäure und Zitronensäure bevorzugt. Gegebenenfalls können auch Gemische der genannten Säuren eingesetzt werden, insbesondere in Fällen von Säuren, die neben ihren Säuerungseigenschaften auch andere Eigenschaften, z.B. als Geschmackstoffe, Antioxidantien oder Komplexbildner besitzen, wie beispielsweise Zitronensäure oder Ascorbinsäure. Erfindungsgemäß besonders bevorzugt wird Salzsäure zur Einstellung des pH-Werts verwendet.

In diesen Formulierungen kann gegebenenfalls auf den Zusatz von Editinsäure (EDTA) oder einem der bekannten Salze davon, Natriumedetat, als Stabilisator oder Komplexbildner verzichtet werden. Andere Ausführungsformen beinhalten diese Verbindung(en). In einer solchen bevorzugten Ausführungsform liegt der Gehalt bezogen auf Natriumedetat unter 100 mg / 100 ml, bevorzugt unter 50 mg/ 100ml, besonders bevorzugt unter 20 mg/ 100ml. Generell sind solche Inhalationslösungen bevorzugt, in denen der Gehalt an Natriumedetat bei 0 bis 10mg/100ml liegt.

Den treibgasfreien Inhaltionslösungen können Co-Solventien und/oder weitere Hilfsstoffe zugesetzt werden. Bevorzugte Co-Solventien sind solche, die Hydroxylgruppen oder andere polare Gruppen enthalten, beispielsweise Alkohole - insbesondere Isopropylalkohol, Glykole - insbesondere Propylenglykol, Polyethylenglykol, Polypropylenglykol, Glykolether, Glycerol, Polyoxyethylenalkohole und Polyoxyethylen-Fettsäureester. Unter Hilfs- und Zusatzstoffen wird in diesem Zusammenhang jeder pharmakologisch verträgliche Stoff verstanden, der kein Wirkstoff ist, aber zusammen mit dem (den) Wirkstoff(en) in dem pharmakologisch geeigneten Lösungsmittel formuliert werden kann, um die qualitativen Eigenschaften der Wirkstoffformulierung zu verbessern. Bevorzugt entfalten diese Stoffe keine oder im Kontext mit der angestrebten Therapie keine nennenswerte oder zumindest keine unerwünschte pharmakologische Wirkung. Zu den Hilfs- und Zusatzstoffen zählen z.B. oberflächenaktive Stoffe, wie z.B. Sojalecithin, Ölsäure, Sorbitanester, wie Polysorbate, Polyvinylpyrrolidon sonstige Stabilisatoren, Komplexbildner, Antioxidantien und/oder Konservierungsstoffe, die die Verwendungsdauer der fertigen Arzneimittelformulierung gewährleisten oder verlängern, Geschmackstoffe, Vitamine und/oder sonstige dem Stand der Technik bekannte Zusatzstoffe. Zu den Zusatzstoffen zählen auch pharmakologisch unbedenkliche Salze wie beispielsweise Natriumchlorid als Isotonantien.

Zu den bevorzugten Hilfsstoffen zählen Antioxidantien, wie beispielsweise Ascorbinsäure, sofern nicht bereits für die Einstellung des pH-Werts verwendet, Vitamin A, Vitamin E, Tocopherole und ähnliche im menschlichen Organismus vorkommende Vitamine oder Provitamine.

Konservierungsstoffe können eingesetzt werden, um die Formulierung vor Kontamination mit Keimen zu schützen. Als Konservierungsstoffe eignen sich die dem Stand der Technik bekannten, insbesondere Cetylpyridiniumchlorid, Benzalkoniumchlorid oder Benzoesäure bzw. Benzoate wie Natriumbenzoat in der aus dem Stand der Technik bekannten Konzentration. Die vorstehend genannten Konservierungsstoffe sind vorzugsweise in Konzentrationen von bis zu 50mg/100ml, besonders bevorzugt zwischen 5 und 20 mg/100ml enthalten.

Bevorzugte Formulierungen enthalten außer dem Lösungsmittel Wasser und dem Wirkstoff 1 nur noch Benzalkoniumchlorid und Natriumedetat.

In einer anderen bevorzugten Ausführungsform wird auf Natriumedetat verzichtet.

Die Dosierung der erfindungsgemäßen Verbindungen ist naturgemäß stark von der Applikationsart und der zu therapierenden Erkrankung abhängig. Bei inhalativer Applikation zeichnen sich die Verbindungen der Formel **1** bereits bei Dosen im µg-Bereich durch eine hohe Wirksamkeit aus. Auch oberhalb des µg-Bereichs, lassen sich die Verbindungen der Formel **1** sinnvoll einsetzen. Die Dosierung kann dann beispielsweise auch im Grammbereich liegen. Insbesondere bei nicht inhalativer Applikation können die erfindungsgemäßen Verbindungen mit höherer Dosierung appliziert werden (beispielsweise, aber nicht limitierend im Bereich von 1 bis 1000mg).

Die nachfolgenden Formulierungsbeipiele illustrieren die vorliegende Erfindung ohne sie jedoch in ihrem Umfang zu beschränken:

Pharmazeutische Formulierungsbeispiele

A)

| Tabletten | pro Tablette |
|---|---|
| Wirkstoff **1** | 100 mg |
| Milchzucker | 140 mg |
| Maisstärke | 240 mg |
| Polyvinylpyrrolidon | 15 mg |
| Magnesiumstearat | 5 mg |
| | 500 mg |

Der feingemahlene Wirkstoff, Milchzucker und ein Teil der Maisstärke werden miteinander vermischt. Die Mischung wird gesiebt, worauf man sie mit einer Lösung von Polyvinylpyrrolidon in Wasser befeuchtet, knetet, feuchtgranuliert und trocknet. Das Granulat, der Rest der Maisstärke und das Magnesiumstearat werden gesiebt und miteinander vermischt. Das Gemisch wird zu Tabletten geeigneter Form und Größe verpreßt.

B)

| Tabletten | pro Tablette |
|---|---|
| Wirkstoff 1 | 80 mg |
| Milchzucker | 55 mg |
| Maisstärke | 190 mg |
| Mikrokristalline Cellulose | 35 mg |
| Polyvinylpyrrolidon | 15 mg |
| Natrium-carboxymethylstärke | 23 mg |
| Magnesiumstearat | 2 mg |
| | 400 mg |

Der feingemahlene Wirkstoff, ein Teil der Maisstärke, Milchzucker, mikrokristalline Cellulose und Polyvinylpyrrolidon werden miteinander vermischt, die Mischung gesiebt und mit dem Rest der Maisstärke und Wasser zu einem Granulat verarbeitet, welches getrocknet und gesiebt wird. Dazu gibt man die Natriumcarboxymethylstärke und das Magnesiumstearat, vermischt und verpreßt das Gemisch zu Tabletten geeigneter Größe.

C) Ampullenlösung

| | |
|---|---|
| Wirkstoff **1** | 50 mg |
| Natriumchlorid | 50 mg |
| Aqua pro inj. | 5 ml |

Der Wirkstoff wird bei Eigen-pH oder gegebenenfalls bei pH 5,5 bis 6,5 in Wasser gelöst und mit Natriumchlorid als Isotonans versetzt. Die erhaltene Lösung wird pyrogenfrei filtriert und das Filtrat unter aseptischen Bedingungen in Ampullen abgefüllt, die anschließend sterilisiert und zugeschmolzen werden. Die Ampullen enthalten 5 mg, 25 mg und 50 mg Wirkstoff.

D) Dosieraerosol

| | |
|---|---|
| Wirkstoff **1** | 0,005 |
| Sorbitantrioleat | 0,1 |
| Monofluortrichlormethan und Difluordichlormethan 2 : 3 | ad 100 |

Die Suspension wird in einen üblichen Aerosolbehälter mit Dosierventil gefüllt. Pro Betätigung werden vorzugsweise 50 µl Suspension abgegeben. Der Wirkstoff kann gewünschtenfalls auch höher dosiert werden (z.B. 0.02 Gew.-%).

E) Lösungen (in mg/100ml)

| | |
|---|---|
| Wirkstoff **1** | 333.3 mg |
| Formoterolfumarat | 333.3 mg |
| Benzalkoniumchlorid | 10.0 mg |
| EDTA | 50.0 mg |
| HCl (1n) | ad pH 3.4 |

Diese Lösung kann in üblicher Art und Weise hergestellt werden.

F) Inhalationpulver

| | |
|---|---|
| Wirkstoff **1** | 6 µg |
| Formoterolfumarat | 6 µg |
| Lactose Monohydrat | ad 25 mg |

Die Herstellung des Inhaltionspulvers erfolgt in üblicher Art und Weise durch Mischen der einzelnen Bestandteile.

G) Inhalationpulver

| | |
|---|---|
| Wirkstoff **1** | 10 µg |
| Lactose Monohydrat | ad 5 mg |

Die Herstellung des Inhaltionspulvers erfolgt in üblicher Art und Weise durch Mischen der einzelnen Bestandteile.

## Patentansprüche

**1.** Verbindungen der allgemeinen Formel **1**

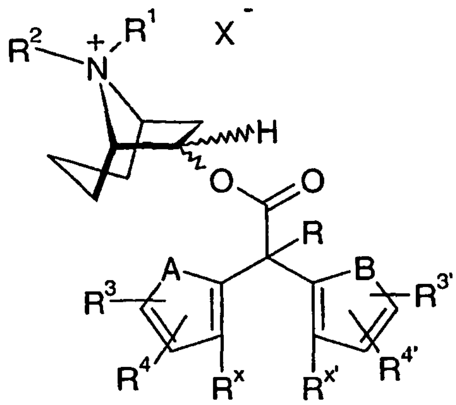

1

worin

| | |
|---|---|
| $X^-$ | ein einfach negativ geladenes Anion, vorzugsweise ein Anion ausgewählt aus der Gruppe bestehend aus Chlorid, Bromid, Iodid, Sulfat, Phosphat, Methansulfonat, Nitrat, Maleat, Acetat, Citrat, Fumarat, Tartrat, Oxalat, Succinat, Benzoat und p-Toluolsulfonat; |
| A und B | gleich oder verschieden, bevorzugt gleich, -O-, -S-, -NH-, $-CH_2-$, -CH=CH-, oder $-N(C_1-C_4-Alkyl)-$; |
| R | Wasserstoff, Hydroxy, $-C_1-C_4$-Alkyl, $-C_1-C_4$-Alkyloxy, $-C_1-C_4$-Alkylen-Halogen, $-O-C_1-C_4$-Alkylen-Halogen, $-C_1-C_4$-Alkylen-OH, $-CF_3$, $CHF_2$, $-C_1-C_4$-Alkylen-$C_1-C_4$-alkyloxy, $-O-COC_1-C_4$-Alkyl, $-O-COC_1-C_4$-Alkylen-Halogen, $-C_1-C_4$-Alkylen-$C_3-C_6$-Cycloalkyl, $-O-COCF_3$ oder Halogen; |
| $R^1$ und $R^2$ | gleich oder verschieden, $-C_1-C_5$-Alkyl, welches gegebenenfalls durch $-C_3-C_6$-Cycloalkyl, Hydroxy oder Halogen substituiert sein kann, oder $R^1$ und $R^2$ gemeinsam eine $-C_3-C_5$-Alkylen-Brücke; |
| $R^3$, $R^4$, $R^{3'}$ und $R^{4'}$, | gleich oder verschieden, Wasserstoff, $C_1-C_4$-Alkyl, $C_1-C_4$-Alkyloxy, Hydroxy, $-CF_3$, $-CHF_2$, CN, $NO_2$ oder Halogen; |
| $R^x$ und $R^{x'}$ | gleich oder verschieden Wasserstoff, $C_1-C_4$-Alkyl, $C_1-C_4$-Alkyloxy, Hydroxy, $-CF_3$, $-CHF_2$, CN, $NO_2$ oder Halogen oder $R^x$ und $R^{x'}$ gemeinsam eine Einfachbindung oder eine verbrückende Gruppe ausgewählt aus den Brücken -O-, -S-, -NH-, $-CH_2-$, $-CH_2-CH_2-$, $-N(C_1-C_4-Alkyl)-$, $-CH(C_1-C_4-Alkyl)-$ und $-C(C_1-C_4-Alkyl)_2-$, bedeuten. |

**2.** Verbindungen der allgemeinen Formel 1 nach Anspruch 1, worin

| | |
|---|---|
| $X^-$ | ein einfach negativ geladenes Anion ausgewählt aus der Gruppe Chlorid, Bromid, 4-Toluolsulfonat und Methansulfonat, bevorzugt Bromid; |
| A und B | gleich oder verschieden, bevorzugt gleich, -O-, -S-, -NH- oder -CH=CH-; |
| R | Wasserstoff, Hydroxy, $-C_1-C_4$-Alkyl, $-C_1-C_4$-Alkyloxy, $-CF_3$, $-CHF_2$, Fluor, Chlor oder Brom; |
| $R^1$ und $R^2$ | gleich oder verschieden, $C_1-C_4$-Alkyl, welches gegebenenfalls durch Hydroxy, Fluor, Chlor oder Brom substituiert sein kann, oder $R^1$ und $R^2$ gemeinsam eine $-C_3-C_4$-Alkylen-Brücke; |
| $R^3$, $R^4$, $R^{3'}$ und $R^{4'}$, | gleich oder verschieden, Wasserstoff, $C_1-C_4$-Alkyl, $C_1-C_4$-Alkyloxy, Hydroxy, $-CF_3$, $-CHF_2$, CN, $NO_2$, Fluor, Chlor oder Brom; |
| $R^x$ und $R^{x'}$ | gleich oder verschieden Wasserstoff, $C_1-C_4$-Alkyl, $C_1-C_4$-Alkyloxy, Hydroxy, $-CF_3$, $-CHF_2$, CN, $NO_2$, Fluor, Chlor oder Brom oder $R^X$ und $R^{X'}$ gemeinsam eine Einfachbindung oder eine verbrückende Gruppe ausgewählt aus den Brücken -O-, -S-, -NH- und $-CH_2-$ bedeuten. |

**3.** Verbindungen der allgemeinen Formel 1 nach Anspruch 1 oder 2, worin

| | |
|---|---|
| $X^-$ | ein einfach negativ geladenes Anion ausgewählt aus der Gruppe Chlorid, Bromid und Methansulfonat, bevorzugt Bromid; |
| A und B | gleich oder verschieden, bevorzugt gleich, -S- oder -CH=CH-; |
| R | Wasserstoff, Hydroxy, Methyl, Ethyl, Methyloxy, Ethyloxy, $-CF_3$, oder Fluor; |
| $R^1$ und $R^2$ | gleich oder verschieden, Methyl, Ethyl, $-CH_2F$ oder $-CH_2-CH_2F$, bevorzugt Methyl oder Ethyl; |
| $R^3$, $R^4$, $R^{3'}$ und $R^{4'}$, | gleich oder verschieden, Wasserstoff, Methyl, Methyloxy, $-CF_3$ oder Fluor; |
| $R^x$ und $R^{x'}$ | gleich oder verschieden Wasserstoff, Methyl, Methyloxy, $-CF_3$ oder Fluor oder $R^x$ und $R^{x'}$ gemeinsam eine Einfachbindung oder die verbrückende Gruppe -O- bedeuten. |

**4.** Verbindungen der allgemeinen Formel **1** nach Anspruch 1, 2 oder 3, worin

| | |
|---|---|
| $X^-$ | ein einfach negativ geladenes Anion ausgewählt aus der Gruppe Chlorid, Bromid und Methansulfonat, bevorzugt Bromid; |
| A und B | gleich oder verschieden, bevorzugt gleich, -S- oder -CH=CH-; |
| R | Wasserstoff, Hydroxy oder Methyl, bevorzugt Hydroxy; |
| $R^1$ und $R^2$ | gleich oder verschieden, Methyl oder Ethyl; |
| $R^3$, $R^4$, $R^{3'}$ und $R^{4'}$, | gleich oder verschieden, Wasserstoff, $-CF_3$ oder Fluor, bevorzugt Wasserstoff; |
| $R^x$ und $R^{x'}$ | gleich oder verschieden Wasserstoff, $-CF_3$ oder Fluor, bevorzugt Wasserstoff oder $R^x$ und $R^{x'}$ gemeinsam eine Einfachbindung oder die verbrückende Gruppe -O-, bevorzugt eine Einfachbindung bedeuten. |

**5.** Verbindungen der allgemeinen Formel 1 nach einem der Ansprüche 1 bis 4, worin

| | |
|---|---|
| $X^-$ | Bromid; |
| A und B | gleich oder verschieden, bevorzugt gleich, -S- oder -CH=CH-; |
| R | Wasserstoff, Hydroxy oder Methyl, bevorzugt Hydroxy; |
| $R^1$ und $R^2$ | Methyl; |
| $R^3$, $R^4$, $R^{3'}$ und $R^{4'}$, | gleich oder verschieden, Wasserstoff oder Fluor, bevorzugt Wasserstoff; |
| $R^x$ und $R^{x'}$ | gleich oder verschieden Wasserstoff oder Fluor, bevorzugt Wasserstoff oder $R^x$ und $R^{x'}$ gemeinsam eine Einfachbindung oder die verbrückende Gruppe -O-, bevorzugt eine Einfachbindung bedeuten. |

**6.** Verbindungen der allgemeinen Formel **1** nach einem der Ansprüche 1 bis 5, gegebenenfalls in Form der einzelnen optischen Isomeren, Mischungen der einzelnen Enantiomeren oder Racemate sowie gegebenenfalls in Form ihrer pharmakologisch verträglichen Säureadditionssalze.

**7.** Verwendung einer Verbindung der allgemeinen Formel **1** gemäß einem der Ansprüche 1 bis 6 als Arzneimittel.

**8.** Verwendung einer Verbindung der allgemeinen Formel **1** gemäß einem der Ansprüche 1 bis 6 zur Herstellung eines Arzneimittels zur Behandlung von Erkrankungen, in denen Anticholinergika einen therapeutischen Nutzen entfalten können.

**9.** Verwendung einer Verbindung der allgemeinen Formel **1** gemäß einem der Ansprüche 1 bis 6 zur Herstellung eines Arzneimittels zur Behandlung von Asthma, COPD, vagal bedingter Sinusbradykardien, Herz-Rhythmus-Störungen, Spasmen im Gastrointestinaltrakt, Spasmen in harnableitenden Wegen und Menstruationsbeschwerden.

**10.** Pharmazeutische Zubereitungen, enthaltend als Wirkstoff eine oder mehrere Verbindungen der allgemeinen Formel **1** gemäß einem der Ansprüche 1 bis 6 oder deren physiologisch verträgliche Salze gegebenenfalls in Kombination mit üblichen Hilfs- und/oder Trägerstoffen.

**11.** Pharmazeutische Zubereitungen nach Anspruch 10, **dadurch gekennzeichnet daß** diese neben einer oder mehrerer der Verbindungen der Formel **1** ferner wenigstens einen weiteren Wirkstoff enthalten, der ausgewählt ist aus

der Gruppe der Betamimetica, Antiallergika, Dopaminagonisten, PAF-Antagonisten, PDE IV-Inhibitoren, Leukotrien-Antagonisten, p38 Kinase-Inhibitoren, EGFR-Kinase-Hemmer und Corticosteroide, sowie Wirkstoffkombinationen davon.

**12.** Zwischenprodukte der allgemeinen Formel 4

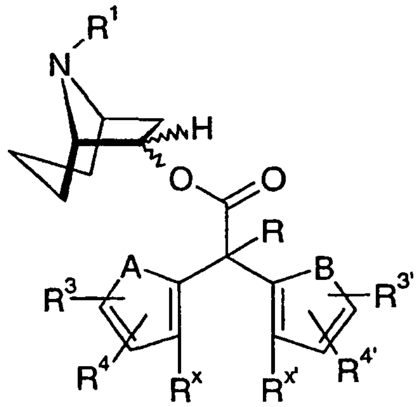

4

worin die Reste A, B, R, $R^1$, $R^3$, $R^{3'}$, $R^4$, $R^{4'}$, $R^X$ und $R^{x'}$ die in den Ansprüchen 1 bis 5 genannten Bedeutungen haben können., gegebenenfalls in Form ihrer Säureadditionssalze.

**13.** Verwendung der Verbindungen der allgemeinen Formel 2

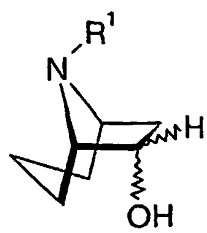

2

gegebenenfalls in Form ihrer Säureadditionssalze, worin $R^1$ die in den Ansprüchen 1 bis 5 genannten Bedeutungen haben kann, zur Herstellung von Verbindungen der Formel 4 nach Anspruch 12.

## Claims

**1.** Compounds of general formula 1

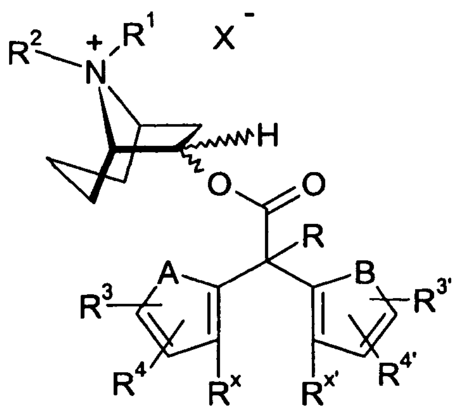

**1**

wherein

| | |
|---|---|
| $X^-$ | denotes an anion with a single negative charge, preferably an anion selected from the group consisting of chloride, bromide, iodide, sulphate, phosphate, methanesulphonate, nitrate, maleate, acetate, citrate, fumarate, tartrate, oxalate, succinate, benzoate and p-toluenesulphonate; |
| A and B | which may be identical or different, preferably identical, denote -O-, -S-, -NH-, $-CH_2-$, -CH=CH-, or $-N(C_1-C_4\text{-alkyl})-$; |
| R | denotes hydrogen, hydroxy, $-C_1-C_4$-alkyl, $-C_1-C_4$-alkyloxy, $-C_1-C_4$-alkylene-halogen, $-O-C_1-C_4$-alkylene-halogen, $-C_1-C_4$-alkylene-OH, $-CF_3$, $CHF_2$, $-C_1-C_4$-alkylene-$C_1-C_4$-alkyloxy, $-O-COC_1-C_4$-alkyl, $-O-COC_1-C_4$-alkylene-halogen, $-C_1-C_4$-alkylene-$C_3-C_6$-cycloalkyl, $-O-COCF_3$ or halogen; |
| $R^1$ and $R^2$ | which may be identical or different denote $-C_1-C_5$-alkyl, which may optionally be substituted by $-C_3-C_6$-cycloalkyl, hydroxy or halogen,<br>or<br>$R^1$ and $R^2$ together denote a $-C_3-C_5$-alkylene bridge; |
| $R^3$, $R^4$, $R^{3'}$ and $R^{4'}$, | which may be identical or different, denote hydrogen, $C_1-C_4$-alkyl, $C_1-C_4$-alkyloxy, hydroxy, $-CF_3$, $-CHF_2$, CN, $NO_2$ or halogen; |
| $R^x$ and $R^{x'}$ | which may be identical or different denote hydrogen, $C_1-C_4$-alkyl, $C_1-C_4$-alkyloxy, hydroxy, $-CF_3$, $-CHF_2$, CN, $NO_2$ or halogen<br>or<br>$R^x$ and $R^{x'}$ together denote a single bond or a bridging group selected from the bridges -O-, -S-, -NH-, $-CH_2-$, $-CH_2-CH_2-$, $-N(C_1-C_4\text{-alkyl})-$, $-CH(C_1-C_4\text{-alkyl})-$ and $-C(C_1-C_4\text{-alkyl})_2-$. |

**2.** Compounds of general formula **1** according to claim 1, wherein

| | |
|---|---|
| $X^-$ | denotes an anion with a single negative charge selected from among chloride, bromide, 4-toluenesulphonate and methanesulphonate, preferably bromide; |
| A and B | which may be identical or different, preferably identical, denote -O-, -S-, -NH- or -CH=CH-; |
| R | denotes hydrogen, hydroxy, $-C_1-C_4$-alkyl, $-C_1-C_4$-alkyloxy, $-CF_3$, $-CHF_2$, fluorine, chlorine or bromine; |
| $R^1$ and $R^2$ | which may be identical or different, denote $C_1-C_4$-alkyl, which may optionally be substituted by hydroxy, fluorine, chlorine or bromine, or<br>$R^1$ and $R^2$ together denote a $-C_3-C_4$-alkylene bridge; |
| $R^3$, $R^4$, $R^{3'}$ and $R^{4'}$, | which may be identical or different, denote hydrogen, $C_1-C_4$-alkyl, $C_1-C_4$-alkyloxy, hydroxy, $-CF_3$, $-CHF_2$, CN, $NO_2$, fluorine, chlorine or bromine; |
| $R^x$ and $R^{x'}$ | which may be identical or different denote hydrogen, $C_1-C_4$-alkyl, $C_1-C_4$-alkyloxy, hydroxy, $-CF_3$, $-CHF_2$, CN, $NO_2$, fluorine, chlorine or bromine<br>or<br>$R^x$ and $R^{x'}$ together denote a single bond or a bridging group selected from the bridges -O-, -S-, -NH- and $-CH_2-$. |

**3.** Compounds of general formula **1** according to claim 1 or 2, wherein

| | |
|---|---|
| $X^-$ | denotes an anion with a single negative charge selected from among chloride, bromide and methanesulphonate, preferably bromide; |
| A and B | which may be identical or different, preferably identical, denote -S- or -CH=CH-; |
| R | denotes hydrogen, hydroxy, methyl, ethyl, methyloxy, ethyloxy, $-CF_3$, or fluorine; |
| $R^1$ and $R^2$ | which may be identical or different denote methyl, ethyl, $-CH_2F$ or $-CH_2-CH_2F$, preferably methyl or ethyl; |
| $R^3$, $R^4$, $R^{3'}$ and $R^{4'}$, | which may be identical or different, denote hydrogen, methyl, methyloxy, $-CF_3$ or fluorine; |
| $R^X$ and $R^{x'}$ | which may be identical or different denote hydrogen, methyl, methyloxy, $-CF_3$ or fluorine or $R^X$ and $R^{X'}$ together denote a single bond or the bridging group -O-. |

**4.** Compounds of general formula **1** according to claim 1, 2 or 3, wherein

| | |
|---|---|
| $X^-$ | denotes an anion with a single negative charge selected from among chloride, bromide and methanesulphonate, preferably bromide; |
| A and B | which may be identical or different, preferably identical, denote -S- or -CH=CH-; |
| R | denotes hydrogen, hydroxy or methyl, preferably hydroxy; |
| $R^1$ and $R^2$ | which may be identical or different denote methyl or ethyl; |
| $R^3$, $R^4$, $R^{3'}$ | and $R^{4'}$, which may be identical or different, denote hydrogen, $-CF_3$ or fluorine, preferably hydrogen; |
| $R^x$ and $R^{x'}$ | which may be identical or different denote hydrogen, $-CF_3$ or fluorine, preferably hydrogen or $R^x$ and $R^{x'}$ together denote a single bond or the bridging group -O-, preferably a single bond. |

**5.** Compounds of general formula **1** according to one of claims 1 to 4, wherein

| | |
|---|---|
| $X^-$ | denotes bromide; |
| A and B | which may be identical or different, preferably identical, denote -S- or -CH=CH-; |
| R | denotes hydrogen, hydroxy or methyl, preferably hydroxy; |
| $R^1$ and $R^2$ | denotes methyl; |
| $R^3$, $R^4$, $R^{3'}$ and $R^{4'}$, | which may be identical or different, denote hydrogen or fluorine, preferably hydrogen; |
| $R^x$ and $R^{x'}$ which | may be identical or different denote hydrogen or fluorine, preferably hydrogen or $R^x$ and $R^{x'}$ together denote a single bond or the bridging group -O-, preferably a single bond. |

**6.** Compounds of general formula **1** according to one of claims 1 to 5, optionally in the form of the individual optical isomers, mixtures of the individual enantiomers or racemates as well as optionally in the form of the pharmacologically acceptable acid addition salts thereof.

**7.** Use of a compound of general formula **1** according to one of claims 1 to 6 as a pharmaceutical composition.

**8.** Use of a compound of general formula **1** according to one of claims 1 to 6 for preparing a pharmaceutical composition for the treatment of diseases in which anticholinergics can develop a therapeutic benefit.

**9.** Use of a compound of general formula **1** according to one of claims 1 to 6 for preparing a pharmaceutical composition for the treatment of asthma, COPD, vagally induced sinus bradycardia, heart rhythm disorders, spasms in the gastrointestinal tract, spasms in the urinary tract and menstrual pain.

**10.** Pharmaceutical preparations, containing as active substance one or more compounds of general formula **1** according to one of claims 1 to 6 or the physiologically acceptable salts thereof optionally in combination with conventional excipients and/or carriers.

**11.** Pharmaceutical preparations according to claim 10, **characterised in that** they contain, in addition to one or more of the compounds of formula **1,** at least one other active substance which is selected from among the betamimetics, antiallergics, dopamine agonists, PAF antagonists, PDE-IV inhibitors, leukotriene antagonists, p38 kinase inhibitors, EGFR kinase inhibitors and corticosteroids as well as combinations of active substances thereof.

**12.** Intermediate products of general formula 4

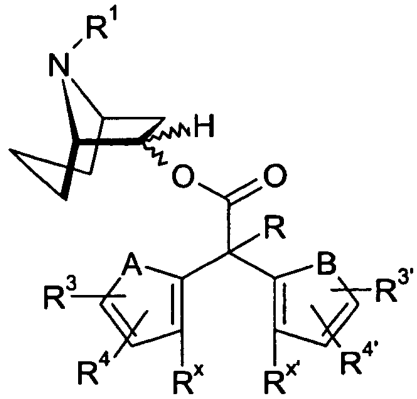

4

wherein the groups A, B, R, $R^1$, $R^3$, $R^{3'}$, $R^4$, $R^{4'}$, $R^x$ and $R^{x'}$ may have the meanings given in claims 1 to 5, optionally in the form of the acid addition salts thereof.

**13.** Use of the compounds of general formula 2

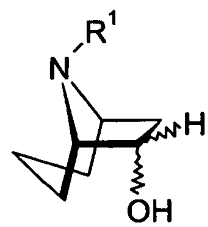

2

optionally in the form of the acid addition salts thereof, wherein $R^1$ may have the meanings given in claims 1 to 5, for preparing compounds of formula 4 according to claim 12.

**Revendications**

**1.** Composés de formule générale 1

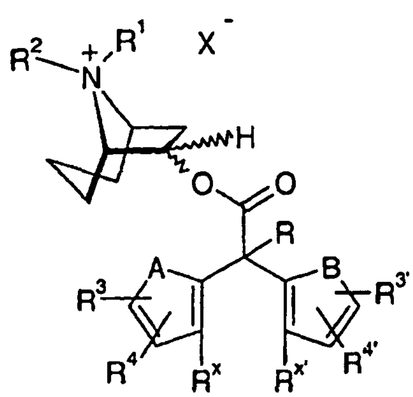

1

où

$X^-$ représente un anion à une charge négative, de préférence un anion choisi dans le groupe consistant en chlorure,

bromure, iodure, sulfate, phosphate, méthanesulfonate, nitrate, maléate, acétate, citrate, fumarate, tartrate, oxalate, succinate, benzoate et p-toluènesulfonate ;
A et B, identiques ou différents, de préférence identiques, représentent -O-, -S-, -NH-, $-CH_2-$, -CH=CH-, ou -N($C_1$-$C_4$-alkyle)- ;
R représente l'hydrogène, hydroxyle, -$C_1$-$C_4$-alkyle, -$C_1$-$C_4$-alkyloxy, -$C_1$-$C_4$-alkylène-halogène, -O-$C_1$-$C_4$-alkylène-halogène, -$C_1$-$C_4$-alkylène-OH, $-CF_3$, $CHF_2$, -$C_1$-$C_4$-alkylène-$C_1$-$C_4$-alkyloxy, -O-COC$_1$-$C_4$-alkyle, -O-COC$_1$-$C_4$-alkylène-halogène, -$C_1$-$C_4$-alkylène-$C_3$-$C_6$-cycloalkyle, $-O-COCF_3$ ou halogène ;
$R^1$ et $R^2$, identiques ou différents, représentent -$C_1$-$C_5$-alkyle, qui peut éventuellement être substitué par -$C_3$-$C_6$-cycloalkyle, hydroxyle ou halogène,
ou
$R^1$ et $R^2$ représentent ensemble un pont -$C_3$-$C_5$-alkylène ;
$R^3$, $R^4$, $R^{3'}$ et $R^{4'}$, identiques ou différents, représentent l'hydrogène, $C_1$-$C_4$-alkyle, $C_1$-$C_4$-alkyloxy, hydroxyle, $-CF_3$, $-CHF_2$, CN, $NO_2$ ou halogène ;
$R^x$ et $R^{x'}$, identiques ou différents, représentent l'hydrogène, $C_1$-$C_4$-alkyle, $C_1$-$C_4$-alkyloxy, hydroxyle, $-CF_3$, $-CHF_2$, CN, $NO_2$ ou halogène
ou
$R^x$ et $R^{x'}$ représentent ensemble une simple liaison ou un groupe de pontage choisi parmi les ponts -O-, -S-, -NH-, $-CH_2-$, $-CH_2-CH_2-$, -N($C_1$-$C_4$-alkyle)-, -CH($C_1$-$C_4$-alkyle)- et -C($C_1$-$C_4$-alkyle)$_2$-.

**2.** Composés de formule générale 1 selon la revendication 1
où
$X^-$ représente un anion à une charge négative choisi dans le groupe chlorure, bromure, 4-toluènesulfonate et méthanesulfonate, de préférence bromure ;
A et B, identiques ou différents, de préférence identiques, représentent -O-, -S-, -NH- ou -CH=CH- ;
R représente l'hydrogène, hydroxyle, -$C_1$-$C_4$-alkyle, -$C_1$-$C_4$-alkyloxy, $-CF_3$, $-CHF_2$, fluor, chlore ou brome ;
$R^1$ et $R^2$, identiques ou différents, représentent -$C_1$-$C_4$-alkyle, qui peut éventuellement être substitué par hydroxyle, fluor, chlore ou brome,
ou
$R^1$ et $R^2$ représentent ensemble un pont -$C_3$-$C_4$-alkylène ;
$R^3$, $R^4$, $R^{3'}$ et $R^{4'}$, identiques ou différents, représentent l'hydrogène, $C_1$-$C_4$-alkyle, $C_1C_4$-alkyloxy, hydroxyle, $-CF_3$, $-CHF_2$, CN, $NO_2$, fluor, chlore ou brome ;
$R^x$ et $R^{x'}$, identiques ou différents, représentent l'hydrogène, $C_1$-$C_4$-alkyle, $C_1$-$C_4$-alkyloxy, hydroxyle, $-CF_3$, $-CHF_2$, CN, $NO_2$, fluor, chlore ou brome
ou
$R^x$ et $R^{x'}$ représentent ensemble une simple liaison ou un groupe de pontage choisi parmi les ponts -O-, -S-, -NH- et $-CH_2-$.

**3.** Composés de formule générale 1 selon la revendication 1 ou 2
où
$X^-$ représente un anion à une charge négative choisi dans le groupe chlorure, bromure et méthanesulfonate, de préférence bromure ;
A et B, identiques ou différents, de préférence identiques, représentent -S- ou -CH=CH- ;
R représente l'hydrogène, hydroxyle, méthyle, éthyle, méthyloxy, éthyloxy, $-CF_3$ ou fluor ;
$R^1$ et $R^2$, identiques ou différents, représentent méthyle, éthyle, $-CH_2F$ ou $-CH_2-CH_2F$, de préférence méthyle ou éthyle ;
$R^3$, $R^4$, $R^{3'}$ et $R^{4'}$, identiques ou différents, représentent l'hydrogène, méthyle, méthyloxy, $-CF_3$ ou fluor ;
$R^x$ et $R^{x'}$, identiques ou différents, représentent l'hydrogène, méthyle, méthyloxy, $-CF_3$ ou fluor
ou
$R^x$ et $R^{x'}$ représentent ensemble une simple liaison ou le groupe de pontage -O-.

**4.** Composés de formule générale 1 selon la revendication 1, 2 ou 3
où
X- représente un anion à une charge négative choisi dans le groupe chlorure, bromure et méthanesulfonate, de préférence bromure ;
A et B, identiques ou différents, de préférence identiques, représentent -S- ou -CH=CH- ;
R représente l'hydrogène, hydroxyle ou méthyle, de préférence hydroxyle ;
$R^1$ et $R^2$, identiques ou différents, représentent méthyle ou éthyle ;
$R^3$, $R^4$, $R^{3'}$ et $R^{4'}$, identiques ou différents, représentent l'hydrogène, $-CF_3$ ou fluor, de préférence l'hydrogène ;

$R^x$ et $R^{x'}$, identiques ou différents, représentent l'hydrogène, $-CF_3$ ou fluor, de préférence l'hydrogène ou $R^x$ et $R^{x'}$ représentent ensemble une simple liaison ou le groupe de pontage -O-, de préférence une simple liaison.

**5.** Composés de formule générale 1 selon l'une des revendications 1 à 4
où
$X^-$ représente bromure ;
A et B, identiques ou différents, de préférence identiques, représentent -S- ou -CH=CH- ;
R représente l'hydrogène, hydroxyle ou méthyle, de préférence hydroxyle ;
$R^1$ et $R^2$ représentent méthyle ;
$R^3$, $R^4$, $R^{3'}$ et $R^{4'}$, identiques ou différents, représentent l'hydrogène ou fluor, de préférence l'hydrogène ;
$R^x$ et $R^{x'}$, identiques ou différents, représentent l'hydrogène ou fluor, de préférence l'hydrogène
ou
$R^x$ et $R^{x'}$ représentent ensemble une simple liaison ou le groupe de pontage -O-, de préférence une simple liaison.

**6.** Composés de formule générale 1 selon l'une des revendications 1 à 5, éventuellement sous forme des différents isomères optiques, de mélanges des différents énantiomères ou de racémates, ainsi éventuellement que sous forme de leurs sels d'addition d'acide pharmacologiquement acceptables.

**7.** Utilisation d'un composé de formule générale 1 selon l'une des revendications 1 à 6 comme médicament.

**8.** Utilisation d'un composé de formule générale 1 selon l'une des revendications 1 à 6 pour la production d'un médicament pour le traitement des maladies dans lesquelles les anticholinergiques peuvent présenter une utilité thérapeutique.

**9.** Utilisation d'un composé de formule générale 1 selon l'une des revendications 1 à 6 pour la production d'un médicament pour le traitement de l'asthme, de la COPD, des bradycardies sinusales d'origine vagale, des troubles du rythme cardiaque, des spasmes dans les voies gastro-intestinales, des spasmes dans les voies urinaires et des troubles de menstruation.

**10.** Préparations pharmaceutiques contenant comme principe actif un ou plusieurs composés de formule générale 1 selon l'une des revendications 1 à 6 ou leurs sels physiologiquement acceptables éventuellement en combinaison avec des adjuvants et/ou supports courants.

**11.** Préparations pharmaceutiques selon la revendication 10 **caractérisées en ce qu'**elles contiennent, outre un ou plusieurs des composés de formule 1, au moins un autre principe actif qui est choisi dans le groupe des bêtamimétiques, des antiallergiques, des agonistes de dopamine, des antagonistes de PAF, des inhibiteurs de PDE IV, des antagonistes de leucotriènes, des inhibiteurs de kinase p38, des inhibiteurs de kinase EGFR et des corticostéroïdes, ainsi que leurs combinaisons de principes actifs.

**12.** Produits intermédiaires de formule générale 4

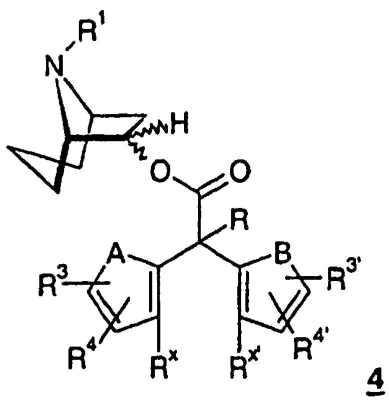

**4**

où les groupements A, B, R, $R^1$, $R^3$, $R^{3'}$, $R^4$, $R^{4'}$, $R^x$ et $R^{x'}$ peuvent avoir les significations citées dans les revendications 1 à 5, éventuellement sous forme de leurs sels d'addition d'acide.

**13.** Utilisation des composés de formule générale 2

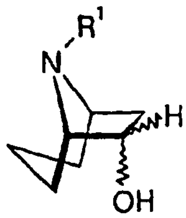

2

éventuellement sous forme de leurs sels d'addition d'acide, où $R^1$ peut avoir les significations citées dans les revendications 1 à 5, pour la production de composés de formule 4 selon la revendication 12.